# EUROPÄISCHE PATENTANMELDUNG

(11) **EP 3 375 872 A1**
(43) Veröffentlichungstag der Anmeldung: **19.09.2018**
(21) Anmeldenummer: 18161541.0
(22) Anmeldetag: 12.06.2008
(51) Int. Cl.: C12N 9/64, A61K 38/48, C07K 14/705

(54) **ACE2 POLYPEPTID**

(30) Priorität: 12.06.2007 AT 9132007; 08.04.2008 EP 08450052
(62) Teilanmeldung aus: 12165189.7
(71) Anmelder: Apeiron Biologics AG, 1030 Wien (AT)
(72) Erfinder: SCHUSTER, Manfred, 2191 Schrick (AT); LOIBNER, Hans, 1230 Wien (AT); JANZEK-HAWLAT, Evelyne, 1230 Wien (AT); PEBALL, Bernhard, 1070 Wien (AT); STRANNER, Stefan, 1230 Wien (AT); WAGNER, Bettina, 2700 Wiener Neustadt (AT); WEIK, Robert, 2724 Hohe Wand-Stollhof (AT)
(74) Vertreter: Jewson, Rie

(57) **Zusammenfassung**

Die vorliegende Erfindung betrifft rekombinantes ACE2 Polypeptid, wobei das ACE2 Polypetid als Dimer vorliegt. Das Dimer wird im speziellen aus glykosylierten Monomeren gebildet und zur Herstellung pharmazeutischer Produkte mit verlängerter Halbwertszeit eingesetzt.

## Beschreibung

Die vorliegende Erfindung betrifft das Gebiet der Produktion rekombinanter Proteine.

Angiotensin Converting Enzyme (ACE2) stellt ein Schlüsselenzym des Renin-Angiotensin-Systems dar. Als Carboxypeptidase wird es membranverankert, als Rezeptor vor allem auf Lungen-, Nieren- und Herzzellen, aber auch auf Endothelzellen exprimiert und spaltet diverse Peptidsubstrate. Prominente Substratvertreter sind Angiotensin II (Ang II), welches zu Angiotensin 1-7 (Ang 1-7) gespalten wird, Angiotensin-I, welches zu Angiotensin 1-9 gespalten wird, aber ebenso Apelin und Bradykinin. Ang II und Ang 1-7 sind Antagonisten des Renin-Angiotensin-Systems. ACE2 ist über die Steuerung der Peptidverhältnisse maßgeblich für die Regulation der Gefäßdicke sowie für die Endothelpermeabilität verantwortlich und beeinflusst hierbei die Homeostase des Organismus. Die Expression von ACE2 ist u.a. zytokingesteuert und wird in diversen entzündlichen Erkrankungen herabgesetzt, was in weiterer Folge zu einer pathologischen Anreicherung von Ang II, einem der wichtigsten Substrate von ACE2, führt.

ACE2 dient zur Behandlung von ARDS oder ALI, zwei Formen einer akuten Lungenerkrankung, mit welchen eine herunterregulierte ACE2 Expression in der Lunge einhergeht. Für diese Therapie wird rekombinantes lösliches humanes ACE2 verwendet, welches systemisch gegeben wird und binnen kürzester Zeit im gesamten Organismus zur Verfügung steht, um eine erhöhte Ang II Konzentration abzubauen und dabei Ang 1-7 zu erzeugen. Dies kompensiert die negativen Auswirkungen erhöhter Ang II Konzentrationen. Hierfür ist es wünschenswert ein Produkt in Händen zu haben, das ein geeignetes pharmakologisches Profil aufweist: Dementsprechend kennzeichnet sich eine geeignete Substanz durch eine gute Verteilung im Organismus, eine pharmakologisch sinnvolle Halbwertszeit sowie durch eine geringe Immunogenizität. Insbesondere muss das Produkt enzymatisch aktiv, gut löslich sowie auch in Lösung stabil sein und sich in hoher Reinheit reproduzierbar und ökonomisch darstellen lassen.

Tipnis et al. (J Biol Chem. 275 (43) (2000): 33238-43) beschreibt die Isolierung von ACE2 (hier noch als ACEH bezeichnet) und seine cDNA-Isolierung. Durch Produktion in CHO-Zellen wurde ein glykosyliertes Protein-Monomer mit einer Molmasse von 120 kDa erhalten. Nach Deglykoslierung wies dieses Protein eine Masse von 85 kDa auf.

Donoghue (Circ Res. 87 (5) (2000): 1-9) beschreibt die Expression von löslichem ACE2 in CHO-Zellen, welches nicht voll glykosyliert ist und mit einer Molmasse von ca. 90 kDa charakterisiert wurde. Dieses Dokument betrifft des Weiteren Sequenzvergleiche diverser ACE2-Sequenzen und Anti-ACE2 -Antikörper.

WO 2004/023270 A2 betrifft die Kristallisierung von ACE2 nach der Expression von ACE2-Monomeren in Sf9 Insektenzellen. Die Molmasse des Proteins wurde dabei mit 89,6 kDa nach einer Massenspektrometrieanalyse angegeben.

Die therapeutische Umsetzung einer effizienten Enzymsubstitutionstherapie erfordert einen Herstellungsprozess, der ökonomisch und reproduzierbar ein hochreines pharmakologisch effizientes Produkt darstellt. Der lösliche, extrazelluläre Abschnitt von ACE2 enthält 7 N-Glykosylierungsstellen. Nicht-glykosyliertes ACE2 ist schlecht löslich, neigt zur Aggregation, ist vermehrt immunogen und weist eine verkürzte Halbwertszeit auf. Außerdem besitzt es einen kleineren hydrodynamischen Durchmesser, welcher vor allem die Aufreinigung negativ beeinflusst. Es ist daher ein Ziel der vorliegen Erfindung hoch aktives ACE2 mit einer guten in vivo Halbwertszeit zur Verfügung zu stellen. Dieses Ziel wird durch die Gegenstände der Ansprüche erfüllt.

Die vorliegende Erfindung betrifft rekombinantes ACE2 Polypeptid, welches als Dimer vorliegt. Vorzugsweise ist das rekombinantes ACE2 Polypeptid glykosyliert, wobei die Glycogruppen eines ACE2 Polypeptid Monomers in Summe mindestens 10, 11, 12, 13, 14, 15, oder mindestens 16 Sialylsäurereste, vorzugsweise mindestestens 14 Sialylsäurereste, aufweisen und wobei das ACE2 Polypetid als Dimer vorliegt. Ebenso umfasst von der vorliegenden Erfindung ist ein ACE2 Monomer mit den hierin genannten Glykosylierungen, Molgewichten und weiteren Spezifizierungen. Das Dimer ist vorzugsweise ein Homo-Dimer, seine Monomereinheiten haben im Speziellen eine identische Sequenz und Glycosylierung. In der Regel sind die Monomereinheiten des Dimers nicht-kovalent verbunden. Ein Monomer kann aus dem Dimer durch oder denaturierende Schritte ohne Weiteres erhalten werden. Alle hierhin genannten vorzugsweise Glycosylierungen betreffen sowohl das Dimer als auch das Monomer, inklusive eines oder beider Monomere des Dimerkomplexes. Insbesondere bevorzugt enthält das Dimer zwei Zink Ionen.

Unter Sialylsäurereste werden insbesondere Reste vom N-Acetylneuraminsäure-Typ (Neu5Ac) verstanden, im speziellen an N- oder O-Glycosylierungen.

Grundsätzlich ist die Stabilität eines Therapeutikums ein sehr wichtiges Kriterium für dessen Halbwertszeit und somit dessen Wirksamkeit.

Rekombinantes humanes ACE2 wurde bisher ausschließlich als Monomer identifiziert und ist auch als solches in der Literatur hinsichtlich seiner Funktionalität, seiner Kristallstruktur sowie hinsichtlich seiner Interaktion mit einem hochspezifischen Inhibitor beschrieben.

So beschreibt beispielsweise Towler et al. (J Biol Chem 279(17), 2004: 17996-18007) unter Verweis auf Vickers et al. (J Biol Chem 277(17), 2002: 14838-14843) die Expression von ACE2 in Sf9 Insektenzellen, welche nach schonender Größenausschlusschromatographie als letzten Schritt der Aufreinigung als Monomere erhalten wurden. Die Molmasse eines Monomers betrug 89,6 kDa.

Käuflich erwerbbares ACE2 liegt ebenfalls in Monomerform vor (von R&D Systems, Katalognummer 933-ZN, Lot Nummer FIU035071) welches nach dem in Tipnis et al. (2000, oben) genannten Verfahren hergestellt wird, mit dem Unterschied, dass als Expressionssystem NSO anstelle von CHO Zellen verwendet werden. Es werden Monomere beschrieben, die sowohl unter reduzierenden und nicht-reduzierenden Bedingungen ein Molgewicht von 120 kDa aufweisen.

Gemäß Donoghue et al. (Circ Res 87, 2000: e1-e9) wurden ACE2 Monomere in CHO Zellen exprimiert, die nach Sezernierung ein Molekulargewicht von 90 kDa zeigten.

Alle hierin angeführten Dokumente und Veröffentlichungen sind durch Bezugnahme hierin aufgenommen.

Zur Stabilisierung des erfindungsgemäßen Therapeutikums wurde erfindungsgemäß ein Verfahren entwickelt, welches ausschließlich zu stabilen ACE2 Dimeren führt, die sowohl produktionstechnische als auch pharmakologische Vorteile im Vergleich zu ACE2 Monomeren aufweisen.

Die Dimerisierung erbringt die folgenden Vorteile:
- Bessere Löslichkeit und Bio-Verfügbarkeit in physiologischen Lösungen: Bedingt durch die stark geladenen Seitenketten, die im ACE2 Komplex nach außen gerichtet sind, ist das Dimer in ebenfalls geladenen Lösungen (zB. Physiologische Infusionslösungen, Serum, Salzlösungen) besser löslich als das Monomer, das ebenfalls hydrophobe Strukturen nach außen präsentieren müßte.
- Keine Aggregatbildung: Das ACE2 Dimer bildet stabile Komplexe ohne Anlagerung weiterer ACE2 Moleküle zum Dimer.
- Reduzierter Proteaseangriff: Nachdem einzelne Proteinabschnitte und hierbei aller Wahrscheinlichkeit nach der C-terminale Teil in das Innere des Dimers gerichtet sind, wird dieser C-Terminus nicht abgebaut.
- Gesteigerte Halbwertszeit: Durch die geringere Immunogenität, die bessere Löslichkeit und der reduzierten Anfälligkeit für proteolytischen Abbau wird die Halbwertszeit des Proteins erhöht.
- Einfachere Proteinreinigung: Der hydrodynamische Durchmesser des ACE2 Dimers ist aufgrund seiner Struktur und einer ausgeprägten Solvatisierungshülle größer als der errechnete. Deswegen lassen sich ACE2 Dimere hervorragend von klassischen Verunreinigungen aus der Proteinexpression, wie zum Beispiel Serum Albumin (67 kDa) durch Größentrennungssäulen abtrennen.

Vorzugsweise ist das rekombinante ACE2 Polypeptid an mindestens 80% der möglichen N-Glykosylierungspositionen glykosyliert und weist einen Zuckeranteil von größer als 10% (Massen-% des gesamten ACE2) oder 11%, 12%, 13%, 14%, vorzugsweise größer als 15% oder 16%, 17%, 18%, 19%, insbesondere größer als 20 % oder 21%, 22%, 23% 24% oder 25%, auf.

Es wurde ein Produktionsprozess entwickelt, der hochreines und enzymatisch aktives, stark komplex glykosyliertes ACE2 reproduzierbar darstellt. Das Produkt zeichnet sich durch seinen hohen Zuckeranteil (>20 Massen %) und die komplexen, stark verzweigten, teilweise negativ geladenen Zuckerstrukturen aus. Diese wirken sich positiv auf die Löslichkeit, die Bioverfügbarkeit, die enzymatische Aktivität sowie die pharmakologischen Eigenschaften des Produktes aus. Durch die Wahl eines geeigneten Expressionskonstruktes, eines geeigneten Expressionswirtes, einer optimierten Selektionsstrategie, durch ein auf den Zellmetabolismus abgestimmtes Medium sowie durch minutiöse begleitende Klonanalytik und Selektion konnte eine Zelllinie hergestellt werden, die das gewünschte Produkt sezerniert.

ACE2 wurde bereits in Insektenzellen Sf9 und Mauszellen NSO exprimiert. Das Material wurde vor allem in in vitro Versuchen eingesetzt. Es gibt auch Resultate über transiente Expression von ACE2 in CHO Zellen. Bis jetzt wurde noch keine Zelllinie mit prozesstauglicher Produktivität hergestellt. Weiters wurde noch keine dementsprechende Klonselektion hinsichtlich der Produkteigenschaften, im Speziellen in Bezug auf die N-Glykosylierung, durchgeführt.

Die Löslichkeit eines Proteins wird nicht nur durch seine Aminosäuresequenz, sondern auch durch seine Faltung sowie durch post-translationale Modifikationen bestimmt. Es sind vor allem geladene Zuckerstrukturen, die maßgeblich die Löslichkeit eines Proteins steigern und dessen pharmakologisches Profil beeinflussen. So konnte für EPO aufgezeigt werden, dass die Anwesenheit von komplexen verzweigten Glykostrukturen die Halbwertszeit dieses Proteins positiv beinflusst.

Prinzipiell kommen zur rekombinanten Expression von ACE2 verschiedene Expressionssysteme in Frage, wobei prokaryontische Wirtszellen aufgrund fehlender Prozessierung von N-Glykosylierungsstellen nicht weiter getestet wurden. Erfolgreiche eukaryontische ACE2 Expression erfolgte bereits in Sf9 Insektenzellen und NSO Mauszellen.

Vorzugsweise weisen mindestens 70% der glykosylierten N-Glykosylierungspositionen eine Struktur unabhängig voneinander ausgewählt aus den Formeln 1-8: 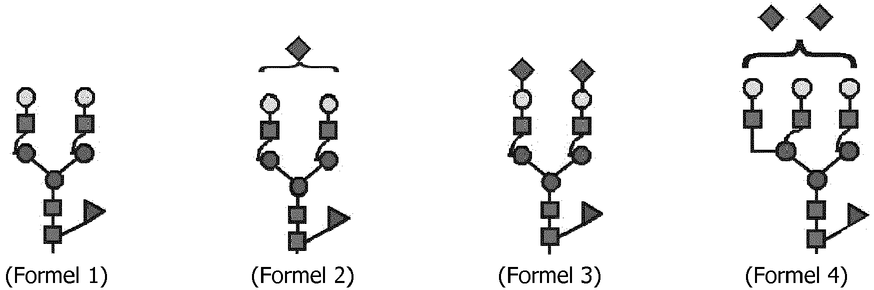 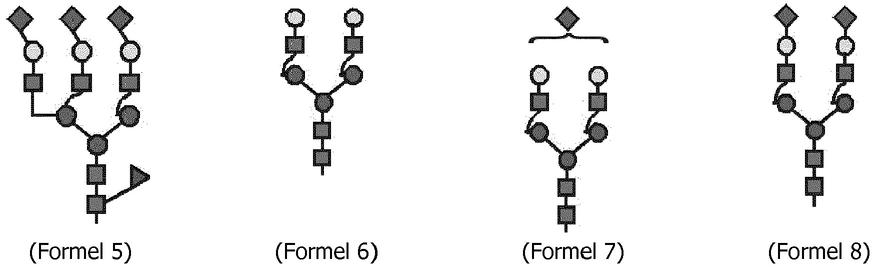 wobei

| | | | |
|---|---|---|---|
| Glc N A c | 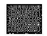 | Fuc | 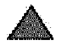 |
| M an | 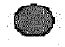 | Xyl | 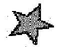 |
| Gal | 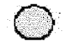 | N eu5Ac | 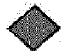 |

ist, auf.

Vorzugsweise sind alle der möglichen N-Glykosylierungspositionen glykosyliert.

Vorzugsweise weisen mindestens 80%, vorzugsweise mindestens 90%, insbesondere 100%, der glykosylierten N-Glykosylierungspositionen eine Struktur der Formeln 1-8 auf.

Vorzugsweise hat ein ACE2 Monomereinheit des Dimers ein Molekulargewicht von mindestens 90 kDa, vorzugsweise mindestens 92 kDa, besonders bevorzugt mindestens 94 kDa, insbesondere mindestens 96 kDa, speziell bevorzugt mindestens 98 kDa, am meisten bevorzugt mindestens 100 kDa, 100,5 kDa, 101 kDa, 101,5 kDa oder auch mindestens 102 kDa. Eine absolute Molmasse - also des Peptids an sich ohne Hydrathülle - kann durch Peptide-map bestimmt werden. Höher glykosylierte Formen können auch Molmassen von mindestens 103 kDa, 104 kDa, 105 kDa, 106 kDa, 107 kDa oder mindestens 108 kDa aufweisen.

Molmassebestimmungen, welche durch weitere Faktoren, wie Hydrathüllen beeinflusst werden - wie z.B. Chromatographien oder Gelelektrophoresen in wässrigen Systemen - können auch zu höheren Ergebnissen führen. In weiteren Ausführungsformen hat eine ACE2 Monomereinheit des Dimers ein scheinbares Molekulargewicht von mindestens 101 kDa oder mindestens 102 kDa, vorzugsweise mindestens 105 kDa, besonders bevorzugt mindestens 109 kDa, insbesondere mindestens 113 kDa, speziell bevorzugt mindestens 117 kDa, am meisten bevorzugt mindestens 119 kDa, wie durch Gelelektrophorese bestimmt wird.

In anderen Ausführungsformen ist das Molekulargewicht des Monomers (scheinbar oder absolut) maximal 102 kDa, 103 kDa, 104 kDa, 108 kDa, 110 kDa, 112 kDa, 116 kDa, 120 kDa, 125 kDa, 130 kDa, 135 kDa oder 140 kDa. Höhere Molekulargewichte sind durch Modifizierungen von ACE2 möglich, z.B. PEGylierung.

Vorzugsweise hat das Monomer (welches kein Dimer bildet) ein Molekulargewicht von mindestens 82 kDa, vorzugsweise mindestens 86 kDa, besonders bevorzugt mindestens 90 kDa, insbesondere mindestens 94 kDa, speziell bevorzugt mindestens 98 kDa, am meisten bevorzugt mindestens 101 kDa bzw. maximal 102 kDa, 103 kDa, 104 kDa, 108 kDa, 110 kDa oder maximal 112 kDa. Diese Molekulargewichte können z.B. nach der Peptide-map-Methode festgestellt werden.

Vorzugsweise weist das ACE2 Polypeptid keine transmembrane Domäne auf. Es handelt sich daher um lösliches ACE2. Besonders bevorzugte Ausführungsformen umfassen dabei lösliche ACE2-Polypeptide, deren Polypeptidkette aus den Aminosäuren 18-740 oder enzymatisch aktiven Fragmenten davon bestehen. Ein weiteres Polypeptid besteht aus den Aminosäuren 18-615 der SEQ ID NO: 1.

Obwohl menschliches ACE2 (SEQ ID NO:1) für die meisten therapeutischen Verwendungen bevorzugt ist, kann auch ACE2 von anderen Säugetieren, z.B. Maus, Ratte, Hamster, Schwein, Primaten oder Rind verwendet werden. ACE2 ist ein universelles Enzym in allen Säugetieren mit dem in verschiedenen Spezies identischen Substrat Ang II. Daher ist es grundsätzlich auch in Fremdorganismen einsetzbar. Somit können mit dem erfindungsgmäßen Protein unabhängig vom Ursprung von ACE2 z.B. Menschen, Mäuse, Ratten, Hamster, Schweine, Primaten oder Rinder behandelt werden. In bevorzugten Ausführungsformen ist jedoch der Ursprung von ACE2 und des behandelten Organismus gleich.

Vorzugsweise ist ein zu Ser740 der SEQ ID NO: 1 (z.B. der C-Terminus) korrespondierendes Serin (oder C-terminale Aminosäure) des ACE2 Polypeptids O-glykosyliert.

Vorzugsweise weisen mind. 70%, vorzugsweise mind. 80%, insbesondere mind. 90%, am meisten bevorzugt 100% der glykosylierten N-Glykosylierungsstellen Sialinsäure auf, vorzugsweise sind die N-Glykosylierungsstellen entsprechend Asn53, Asn90, Asn103, Asn322, Asn432, Asn546, Asn690 der SEQ ID NO:1 sialysiert.

In speziellen Ausführungen ist ein zu Asn53 der SEQ ID NO: 1 korrespondierendes Asparagin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

In speziellen Ausführungen ist ein zu Asn90 der SEQ ID NO: 1 korrespondierendes Asparagin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

In speziellen Ausführungen ist ein zu Asn103 der SEQ ID NO: 1 korrespondierendes Asparagin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

In speziellen Ausführungen ist ein zu Asn322 der SEQ ID NO: 1 korrespondierendes Asparagin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

In speziellen Ausführungen ist ein zu Asn432 der SEQ ID NO: 1 korrespondierendes Asparagin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

In speziellen Ausführungen ist ein zu Asn546 der SEQ ID NO: 1 korrespondierendes Asparagin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

In speziellen Ausführungen ist ein zu Asn690 der SEQ ID NO: 1 korrespondierendes Asparagin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

In speziellen Ausführungen ist ein zu Ser740 der SEQ ID NO: 1 korrespondierendes Serin einfach, zweifach, dreifach oder vierfach sialylisiert. In einer ACE2 Präparation sind vorzugsweise mindestens 50%, 60%, 70%, 80%, 90%, 95%, 99% oder 100% dieser Aminosäure entweder einfach, zweifach, dreifach oder vierfach sialylisiert.

Vorzugsweise weisen mind. 30%, vorzugsweise mind. 40%, insbesondere mind. 55%, am meisten bevorzugt mind. 70% der glykosylierten N-Glykosylierungsstellen mindestens zwei Sialinsäuren auf. In einer ACE2 Präparation sind vorzugsweise mindestens 50%, 60%, 70%, 80%, 90%, 95%, 99% oder 100% dieser Aminosäure derart sialylisiert.

Vorzugsweise ist ein zu Asn53 der SEQ ID NO: 1 korrespondierendes Asparagin N-glykosyliert, vorzugsweise mit einem Glykan der Strukturen nach Formel 1, 2, 3, 4, 5, 6, 7 oder 8.

Vorzugsweise ist ein zu Asn90 der SEQ ID NO: 1 korrespondierendes Asparagin N-glykosyliert, vorzugsweise mit einem Glykan der Strukturen nach Formel 1, 2, 3, 4, 5, 6, 7 oder 8.

Vorzugsweise ist ein zu Asn103 der SEQ ID NO: 1 korrespondierendes Asparagin N-glykosyliert, vorzugsweise mit einem Glykan der Strukturen nach Formel 1, 2, 3, 4, 5, 6, 7 oder 8.

Vorzugsweise ist ein zu Asn322 der SEQ ID NO: 1 korrespondierendes Asparagin N-glykosyliert, vorzugsweise mit einem Glykan der Strukturen nach Formel 1, 2, 3, 4, 5, 6, 7 oder 8.

Vorzugsweise ist ein zu Asn432 der SEQ ID NO: 1 korrespondierendes Asparagin N-glykosyliert, vorzugsweise mit einem Glykan der Strukturen nach Formel 1, 2, 3, 4, 5, 6, 7 oder 8.

Vorzugsweise ist ein zu Asn546 der SEQ ID NO: 1 korrespondierendes Asparagin N-glykosyliert, vorzugsweise mit einem Glykan der Strukturen nach Formel 1, 2, 3, 4, 5, 6, 7 oder 8.

Vorzugsweise ist ein zu Asn690 der SEQ ID NO: 1 korrespondierendes Asparagin N-glykosyliert, vorzugsweise mit einem Glykan der Strukturen nach Formel 1, 2, 3, 4, 5, 6, 7 oder 8.

In einem weiteren Aspekt betrifft die vorliegende Erfindung eine Präparation von rekombinanten ACE2 Polypeptiden, umfassend ein Polypeptid wie hierin definiert (ACE2 Monomere oder Dimere bzw. Monomer-Einheiten der Dimere), wobei der Anteil von ACE2 Polypeptiden mit einem scheinbaren Molekulargewicht, wie durch Gelelektrophorese bestimmbar, von unter 100 kDa oder von unter 101 kDa, bevorzugt von unter 104 kDa, speziell bevorzugt von unter 108 kDa, insbesondere von unter 112 kDa, besonders bevorzugt von unter 117 kDa, am meisten bevorzugt von unter 119 kDa, unter 20%, vorzugsweise unter 10%, insbesondere bevorzugt unter 5%, am meisten bevorzugt unter 1%, im Speziellen bei 0%, liegt, sowie alle Kombinationen hierfür. Z.B. können ACE2 Polypeptide von unter 100 kD zu 0% vorliegen von ACE2 Polypeptide unter 100 kDa unter 20% vertreten sein. Der Anteil bezieht sich auf alle der betroffenen ACE2 Formen und wird hierfür z.B. durch native Gelelektrophorese bestimmt.

Analog hierzu kann als Molmassenmaß die absolute Molmasse dienen welche durch Peptide-map festgestellt werden kann. So ist vorzugsweise der Anteil von ACE2 Polypeptiden mit einem Molekulargewicht von unter 86 kDa, oder von unter 89 kDa, bevorzugt von unter 92 kDa, speziell bevorzugt von unter 94 kDa, insbesondere von unter 97 kDa, besonders bevorzugt von unter 100 kDa, am meisten bevorzugt von unter 101 kDa, unter 20%, vorzugsweise unter 10%, insbesondere bevorzugt unter 5%, am meisten bevorzugt unter 1%, im Speziellen bei 0%, liegt, sowie alle Kombinationen hierfür. Z.B. können ACE2 Polypeptide von unter 86 kD zu 0% vorliegen; von ACE2 Polypeptide unter 97 kDa unter 20% vertreten sein.

Vorzugsweise liegt der Anteil von ACE2 Polypeptiden mit Transmembrandomänen unter 20%, vorzugsweise unter 10%, insbesondere bevorzugt unter 5%, am meisten bevorzugt unter 1%, im Speziellen bei 0%.

Vorzugsweise liegt der Anteil von ACE2 Multimeren unter 20%, vorzugsweise unter 10%, insbesondere bevorzugt unter 5%, am meisten bevorzugt unter 1%, im Speziellen bei 0%. Unter ACE2 Multimeren werden Komplexe mit 3 oder mehr ACE2 Polypeptiden verstanden. In einer Präparation von ACE2 Dimeren liegt zudem vorzugsweise der Anteil von ACE2 Monomeren unter 20%, vorzugsweise unter 10%, insbesondere bevorzugt unter 5%, am meisten bevorzugt unter 1%, im Speziellen bei 0%. In einer Präparation von ACE2 Monomeren liegt zudem vorzugsweise der Anteil von ACE2 Dimeren unter 20%, vorzugsweise unter 10%, insbesondere bevorzugt unter 5%, am meisten bevorzugt unter 1%, im Speziellen bei 0%.

Vorzugsweise beträgt der Anteil an ACE2 Dimeren an ACE2 Molekülen mindestens 10%, 20%, 30%, 40% 50%, 60%, 70%, 80%, 90%, 95% oder mindestens 99%. In weiteren Ausführungsformen kann in Kombination dazu oder unabhängig der Anteil an ACE2 Monomeren an ACE2 Molekülen mindestens 10%, 20%, 30%, 40% 50%, 60%, 70%, 80%, 90%, 95% oder mindestens 99%, sein.

Vorzugsweise ist der Anteil an ACE2 Polypeptiden mit N-glykosyliertem Asparagin entsprechend Asn53 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, und vorzugsweise mit einem Glykan der Strukturen nach Formel 1, 2, 3, 4, 5, 6, 7 oder 8.

Vorzugsweise ist der Anteil an ACE2 Polypeptiden mit N-glykosyliertem Asparagin entsprechend Asn90 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, und vorzugsweise mit einem Glykan der Strukturen nach Formel 1, 2, 3, 4, 5, 6, 7 oder 8.

Vorzugsweise ist der Anteil an ACE2 Polypeptiden mit N-glykosyliertem Asparagin entsprechend Asn103 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, und vorzugsweise mit einem Glykan der Strukturen nach Formel 1, 2, 3, 4, 5, 6, 7 oder 8.

Vorzugsweise ist der Anteil an ACE2 Polypeptiden mit N-glykosyliertem Asparagin entsprechend Asn322 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, und vorzugsweise mit einem Glykan der Strukturen nach Formel 1, 2, 3, 4, 5, 6, 7 oder 8.

Vorzugsweise ist der Anteil an ACE2 Polypeptiden mit N-glykosyliertem Asparagin entsprechend Asn432 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, und vorzugsweise mit einem Glykan der Strukturen nach Formel 1, 2, 3, 4, 5, 6, 7 oder 8.

Vorzugsweise ist der Anteil an ACE2 Polypeptiden mit N-glykosyliertem Asparagin entsprechend Asn546 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, und vorzugsweise mit einem Glykan der Strukturen nach Formel 1, 2, 3, 4, 5, 6, 7 oder 8.

Vorzugsweise ist der Anteil an ACE2 Polypeptiden mit N-glykosyliertem Asparagin entsprechend Asn690 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, und vorzugsweise mit einem Glykan der Strukturen nach Formel 1, 2, 3, 4, 5, 6, 7 oder 8.

Vorzugsweise ist der Anteil an ACE2 Polypeptiden mit O-glykosyliertem Serin entsprechend Ser740 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%.

Vorzugsweise ist die katalytische Aktivität des ACE2 Polypeptids oder der Präparation kkat mindestens 4 /s, vorzugsweise mindestens 5 /s, insbesondere bevorzugt mindesten 6 /s, speziell bevorzugt mindestens 7 /s, am meisten bevorzugt mindestens 7,6 /s, bezogen auf den Ang 1-7 (Angiotensin 1-7) Umsatz. Ang1-7 wird durch ACE2 aus AngII (Angiotensin II) gebildet. Der Umsatz kann wie in den Beispielen beschrieben auf simple Weise getestet werden. Dieser Umsatz bzw. die katalytische Aktivität von ACE2 kann auch aus anderen Assaydaten extrapoliert werden. Die Aktivität kann z.B. wie in der WO 2008/046125 A beschrieben gemessen werden.

In einem weiteren Aspekt betrifft die vorliegende Erfindung ein Verfahren zur Herstellung von rekombinanten ACE2 Polypeptiden oder einer Präparation von rekombinanten ACE2, umfassend die Schritte der Einbringung eines ACE2 codierenden Polynukleotides, vorzugsweise eines ACE2 ohne Transmembrandomäne codierenden Polynukleotids, in eukaryotischen Zellen, Expression des ACE2 Polypeptids und Sammeln des exprimierten ACE2, insbesondere in dimerer Form. Die Zellen können danach selektiert werden, ein erfindungsgemäßes ACE2 Polypeptid, wie hierin beschrieben, insbesondere mit einem hohen Molekulargewicht, zu produzieren.

Vorzugsweise ist das ACE2 codierende Polynukleotid auf einem Vektor vorgesehen.

Vorzugsweise wird die ACE2-Expression mit einem Marker, vorzugsweise DHFR, selektiert. Vorzugsweise liegt der Marker auf dem Vektor vor.

Vorzugsweise weist der Vektor eine IRES für die exprimierte ACE2 mRNA auf (oder codiert hierfür).

Vorzugsweise sind die eukaryotischen Zellen CHO Zellen.

Die vorliegende Erfindung betrifft auch rekombinantes ACE2 Polypeptid oder eine Präparation von rekombinanten ACE2 Polypeptiden erhältlich durch ein solches Verfahren.

In einem weiteren Aspekt liefert die Erfindung eine stabil mit einem ACE2 codierenden Polynukleotid transfizierte eukaryontische Zelllinie (oder Zelle), vorzugsweise CHO Zelllinie (oder Zelle), die ACE2, insbesondere wie oben definiert, exprimiert. Die Zellline kann auf die gewünschten Eigenschaften, wie oben angegeben, z.B. Produktion von Dimeren aus Monomereinheiten mit einem Molekulargewicht von mind. 102 kDa, selektioniert werden.

Die Zelle weist vorzugsweise eine ACE2-Produktivität von mindestens 10 pg/Zelle/Tag, vorzugsweise mindestens 15 pg/Zelle/ Tag, insbesondere bevorzugt von mindestens 20 pg/Zelle/Tag, auf.

Die Expression des ACE2 erfolgt vorzugsweise in Anwesenheit von genügend Zn²⁺-Ionen. Vorzugsweise kommen mindestens 0,5 micromolar, insbesondere bis zu 5 micromolar, Zn²⁺ zur Anwendung, insbesondere kann die Fermentation bei 2,5-3,5 micromolar Zn²⁺ durchgeführt werden. Die Zn²⁺-Konzentration z.B. im Nährmedium der exprimierten Zellen kann mindestens 0,5 µM, 0,75 µM, 1,0 µM, 1,25 µM, 1,5 µM, 1,75 µM, 2,0 µM, 2,25 µM oder mindestens 2,5 µM oder 3,0 µM sein. Weiteren Behandlungsschritte werden vorzugsweise ebenfalls in Anwesenheit von Zn²⁺-Ionen vorgenommen.

In einem weiteren Aspekt betrifft die vorliegende Erfindung Medikamente oder pharmazeutische Präparationen der erfindungsgemäßen ACE2 Produkts. Insbesondere zur Behandlung oder Vorbeugung von Bluthochdruck, Herzinsuffizienzen, wie kongestive, akute oder chronische Herinsuffizienz, Myokardinfarkt oder Artherosklerose, Nierenversagen oder -insuffizienz, Zystennieren (polycistic kidney disease, PKD) oder Lungenerkrankungen wie chronische obstruktive Lungenerkrankung, Pneumonie, Asthma, chronische Bronchitis, Lungenemphysem, zystische Fibrose, interstitielle Lungenerkrankung, pulmonale Hypertonie, Lungenembolie, Lungensarkoidose, Tuberculose, Lungenödem, ALI, ARDS oder Lungenkrebs. Allgemeine Behandlungsindikationen von ACE2 sind z.B. in der WO 2004/000367 A genannt, für die auch das erfindungsgemäße ACE2 Produkt geeignet ist.

Erfindungsgemäß kann eine pharmazeutische Zusammensetzung oder Medikament umfassend das ACE2 Protein zur Verfügung gestellt werden. Solche Zusammensetzungen können pharmazeutisch geeignete Salze derselben, zusätzlich Puffer, Tonizitätskomponenten oder pharmazeutisch geeignete Träger umfassen. Pharmazeutische Träger-Substanzen dienen der besseren Verträglichkeit der Zusammensetzung und ermöglichen bessere Löslichkeit sowie bessere Bioverfügbarkeit der Wirksubstanzen. Beispiele hierfür sind Emulgatoren, Verdickungsmittel, Redoxkomponenten, Stärke, Alkohollösungen, Polyethylenglycol oder Lipide. Die Auswahl eines geeigneten pharmazeutischen Trägers hängt stark von der Art der Verabreichung ab. Für orale Verabreichungen können flüssige oder feste Träger verwendet werden, für Injektionen sind flüssige Endzusammensetzungen erforderlich.

Vorzugsweise umfasst die erfindungsgemäße ACE2 Zusammensetzung Puffersubstanzen oder tonische Substanzen. Mittels Puffer kann der pH-Wert des Medikaments auf physiologische Konditionen eingestellt werden und weiters können pH-Schwankungen abgeschwächt bzw. gepuffert werden. Ein Beispiel hierfür ist ein Phosphatpuffer. Tonische Substanzen dienen zur Einstellung der Osmolarität und können ionische Substanzen, wie zum Beispiel anorganische Salze, wie NaCl oder KCl, oder auch nicht-ionische Substanzen, wie zum Beispiel Glycerin oder Kohlenhydrate, umfassen.

Bevorzugterweise ist die erfindungsgemäß zu verwendende Zusammensetzung oder das Medikament zur systemischen, topischen, oralen oder intranasalen Verabreichung oder als Inhalationsverabreichung geeignet hergerichtet. Diese Verabreichungsformen der Zusammensetzung der vorliegenden Erfindung ermöglichen eine schnelle und unkomplizierte Aufnahme. Sofern die ACE2 Zusammensetzung zur oralen Verabreichung bestimmt ist, wird es vorzugsweise in einer Magensaftresistenten Formulierung oder Verkapselung vorgesehen. Zur oralen Verabreichung können beispielsweise feste bzw. flüssige Medikamente direkt oder aufgelöst bzw. verdünnt eingenommen werden.

Das erfindungsgemäß zu verwendende Medikament ist vorzugsweise zur intravenösen, intraarteriellen, intramuskulären, intravaskulären, intraperitonealen oder subkutanen Verabreichung geeignet hergerichtet. Hierfür eignen sich beispielsweise Injektionen oder Transfusionen. Verabreichungen direkt in die Blutbahn haben den Vorteil, dass die Wirkstoffe des Medikaments im gesamten Körper verteilt werden und die Zielgewebe schnell erreichen.

Vorzugsweise ist die Erfindung wie folgt definiert:
Definition 1. Rekombinantes ACE2 Polypeptid, welches als Dimer vorliegt.
Definition 2. Rekombinantes ACE2 Polypeptid nach Definition 1, dadurch gekennzeichnet, dass es glykosyliert ist, wobei die Glycogruppen eines ACE2 Polypeptid Monomers in Summe mindestens 14 Sialylsäure-Reste aufweisen.
Definition 3. Rekombinantes ACE2 Polypeptid nach Definition 2, dadurch gekennzeichnet, dass es an mindestens 80% der möglichen N-Glykosylierungspositionen glykosyliert ist und einen Zuckeranteil von größer als 10% (Massen-%, bezogen auf das gesamte ACE2-Molekül), vorzugsweise größer als 15%, insbesondere größer als 20 %, aufweist.
Definition 4. Rekombinantes ACE2 Polypeptid nach Definition 1, 2 oder 3, dadurch gekennzeichnet, dass mindestens 70% der glykosylierten N-Glykosylierungspositionen eine Struktur unabhängig voneinander ausgewählt aus den Formeln 1-8: 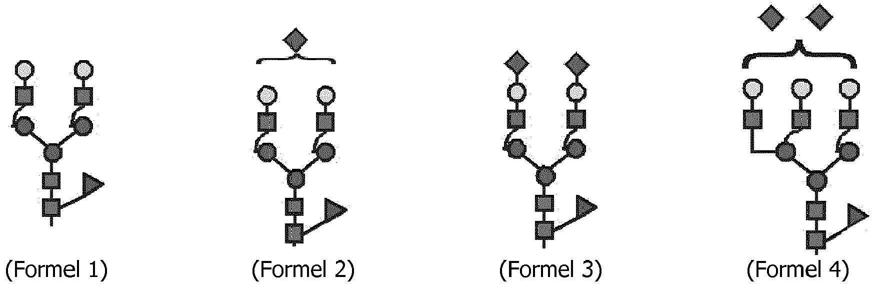 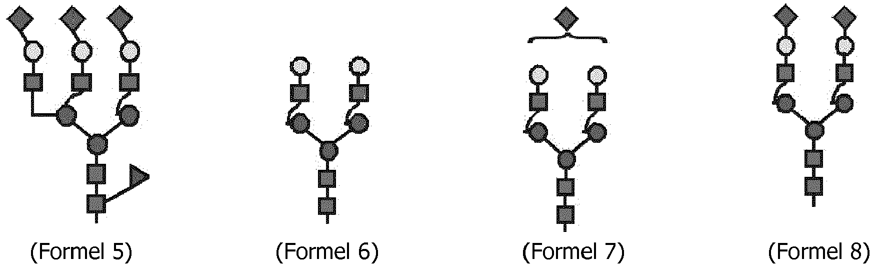 wobei

| | | | |
|---|---|---|---|
| Glc N A c | 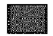 | Fuc | 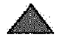 |
| M an | 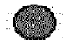 | Xyl | 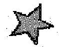 |
| Gal | 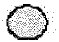 | N eu5Ac | 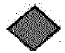 |

ist, aufweisen.
Definition 5. Rekombinantes ACE2 Polypeptid nach einem der Definitionen 1 bis 4, dadurch gekennzeichnet, dass alle der möglichen N-Glykosylierungspositionen glykosyliert sind.
Definition 6. Rekombinantes ACE2 Polypeptid nach einem der Definitionen 1 bis 5, dadurch gekennzeichnet, dass mindestens 80%, vorzugsweise mindestens 90%, insbesondere 100%, der glykosylierten N-Glykosylierungspositionen eine Struktur der Formeln 1-8 aufweisen.
Definition 7. Rekombinantes ACE2 Polypeptid nach einem der Definitionen 1 bis 6, dadurch gekennzeichnet, dass das eine ACE2 Monomereinheit des Dimers ein Molekulargewicht von mindestens 90 kDa, vorzugsweise mindestens 92 kDa, besonders bevorzugt mindestens 94 kDa, insbesondere mindestens 96 kDa, speziell bevorzugt mindestens 98 kDa, am meisten bevorzugt mindestens 101 kDa, aufweist.
Definition 8. Rekombinantes ACE2 Polypeptid nach einem der Definitionen 1 bis 7, dadurch gekennzeichnet, dass das ACE2 Polypeptid keine transmembrane Domäne aufweist, vorzugsweise dass die ACE2 Polypeptidkette aus den Aminosäuren 18-740 von SEQ ID NO: 1 oder enzymatisch aktiven Fragmenten davon besteht.
Definition 9. Rekombinantes ACE2 Polypeptid nach einem der Definitionen 1 bis 8, dadurch gekennzeichnet, dass ein zu Ser740 der SEQ ID NO: 1 korrespondierendes Serin O-glykosyliert ist.
Definition 10. Rekombinantes ACE2 Polypeptid nach einem der Definitionen 1 bis 9, dadurch gekennzeichnet, dass mind. 70%, vorzugsweise mind. 80%, insbesondere mind. 90%, am meisten bevorzugt 100% der glykosylierten N-Glykosylierungsstellen Sialinsäure aufweisen, vorzugsweise sind die N-Glykosylierungsstellen entsprechend Asn53, Asn90, Asn103, Asn322, Asn432, Asn546, Asn690 der SEQ ID NO: 1 sialysiert.
Definition 11. Rekombinantes ACE2 Polypeptid nach einem der Definitionen 1 bis 10, dadurch gekennzeichnet, dass mind. 30%, vorzugsweise mind. 40%, insbesondere mind. 55%, am meisten bevorzugt mind. 70% der glykosylierten N-Glykosylierungsstellen mindestens zwei Sialinsäuren aufweisen.
Definition 12. Rekombinantes ACE2 Polypeptid nach einem der Definitionen 1 bis 11, dadurch gekennzeichnet, dass ein zu Asn53 der SEQ ID NO: 1 korrespondierendes Asparagin N-glykosyliert ist, vorzugsweise mit einem Glykan der Strukturen nach Formel 1, 2, 3, 4, 5, 6, 7 oder 8 wie in Definition 4 definiert.
Definition 13. Rekombinantes ACE2 Polypeptid nach einem der Definitionen 1 bis 12, dadurch gekennzeichnet, dass ein zu Asn90 der SEQ ID NO: 1 korrespondierendes Asparagin N-glykosyliert ist, vorzugsweise mit einem Glykan der Strukturen nach Formel 1, 2, 3, 4, 5, 6, 7 oder 8 wie in Definition 4 definiert.
Definition 14. Rekombinantes ACE2 Polypeptid nach einem der Definitionen 1 bis 13, dadurch gekennzeichnet, dass ein zu Asn103 der SEQ ID NO: 1 korrespondierendes Asparagin N-glykosyliert ist, vorzugsweise mit einem Glykan der Strukturen nach Formel 1, 2, 3, 4, 5, 6, 7 oder 8 wie in Definition 4 definiert.
Definition 15. Rekombinantes ACE2 Polypeptid nach einem der Definitionen 1 bis 14, dadurch gekennzeichnet, dass ein zu Asn322 der SEQ ID NO: 1 korrespondierendes Asparagin N-glykosyliert ist, vorzugsweise mit einem Glykan der Strukturen nach Formel 1, 2, 3, 4, 5, 6, 7 oder 8 wie in Definition 4 definiert.
Definition 16. Rekombinantes ACE2 Polypeptid nach einem der Definitionen 1 bis 15, dadurch gekennzeichnet, dass ein zu Asn432 der SEQ ID NO: 1 korrespondierendes Asparagin N-glykosyliert ist, vorzugsweise mit einem Glykan der Strukturen nach Formel 1, 2, 3, 4, 5, 6, 7 oder 8 wie in Definition 4 definiert.
Definition 17. Rekombinantes ACE2 Polypeptid nach einem der Definitionen 1 bis 16, dadurch gekennzeichnet, dass ein zu Asn546 der SEQ ID NO: 1 korrespondierendes Asparagin N-glykosyliert ist, vorzugsweise mit einem Glykan der Strukturen nach Formel 1, 2, 3, 4, 5, 6, 7 oder 8 wie in Definition 4 definiert.
Definition 18. Rekombinantes ACE2 Polypeptid nach einem der Definitionen 1 bis 17, dadurch gekennzeichnet, dass ein zu Asn690 der SEQ ID NO: 1 korrespondierendes Asparagin N-glykosyliert ist, vorzugsweise mit einem Glykan der Strukturen nach Formel 1, 2, 3, 4, 5, 6, 7 oder 8 wie in Definition 4 definiert.
Definition 19. Präparation von rekombinanten ACE2 Polypeptiden, umfassend ein Polypeptid nach einem der Definitionen 1 bis 18, dadurch gekennzeichnet, dass der Anteil von ACE2 Polypeptiden mit einem Molekulargewicht, bestimmt durch Gelelektrophorese, von unter 100 kDa, bevorzugt von unter 104 kDa, speziell bevorzugt von unter 108 kDa, insbesondere von unter 112 kDa, besonders bevorzugt von unter 117 kDa, am meisten bevorzugt von unter 119 kDa, unter 20%, vorzugsweise unter 10%, insbesondere bevorzugt unter 5%, am meisten bevorzugt unter 1%, im speziellen bei 0%, liegt.
Definition 20. Präparation nach Definition 19, dadurch gekennzeichnet, dass der Anteil von ACE2 Polypeptiden mit Transmembrandomänen unter 20%, vorzugsweise unter 10%, insbesondere bevorzugt unter 5%, am meisten bevorzugt unter 1%, im Speziellen bei 0%, liegt.
Definition 21. Präparation nach Definition 19 oder 20, dadurch gekennzeichnet dass der Anteil von ACE2 Multimeren unter 20%, vorzugsweise unter 10%, insbesondere bevorzugt unter 5%, am meisten bevorzugt unter 1%, im speziellen bei 0%, liegt.
Definition 22. Präparation nach einem der Definitionen 19 bis 21, dadurch gekennzeichnet, dass der Anteil an ACE2 Dimeren an ACE2 Molekülen mindestens 70%, vorzugsweise mindestens 80%, besonders bevorzugt mindestens 90% im speziellen bevorzugt mindestens 95% am meisten bevorzugt mindestens 99%, ist.
Definition 23. Präparation nach einem der Definitionen 19 bis 22, dadurch gekennzeichnet, dass der Anteil an ACE2 Polypeptiden mit N-glykosyliertem Asparagin entsprechend Asn53 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, ist und vorzugsweise mit einem Glykan der Strukturen nach Formel 1, 2, 3, 4, 5, 6, 7 oder 8 wie in Definition 4 definiert.
Definition 24. Präparation nach einem der Definitionen 19 bis 23, dadurch gekennzeichnet, dass der Anteil an ACE2 Polypeptiden mit N-glykosyliertem Asparagin entsprechend Asn90 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, ist und vorzugsweise mit einem Glykan der Strukturen nach Formel 1, 2, 3, 4, 5, 6, 7 oder 8 wie in Definition 4 definiert.
Definition 25. Präparation nach einem der Definitionen 19 bis 24, dadurch gekennzeichnet, dass der Anteil an ACE2 Polypeptiden mit N-glykosyliertem Asparagin entsprechend Asn103 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, ist und vorzugsweise mit einem Glykan der Strukturen nach Formel 1, 2, 3, 4, 5, 6, 7 oder 8 wie in Definition 4 definiert.
Definition 26. Präparation nach einem der Definitionen 19 bis 25, dadurch gekennzeichnet, dass der Anteil an ACE2 Polypeptiden mit N-glykosyliertem Asparagin entsprechend Asn322 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, ist und vorzugsweise mit einem Glykan der Strukturen nach Formel 1, 2, 3, 4, 5, 6, 7 oder 8 wie in Definition 4 definiert.
Definition 27. Präparation nach einem der Definitionen 19 bis 26, dadurch gekennzeichnet, dass der Anteil an ACE2 Polypeptiden mit N-glykosyliertem Asparagin entsprechend Asn432 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, ist und vorzugsweise mit einem Glykan der Strukturen nach Formel 1, 2, 3, 4, 5, 6, 7 oder 8 wie in Definition 4 definiert.
Definition 28. Präparation nach einem der Definitionen 19 bis 27, dadurch gekennzeichnet, dass der Anteil an ACE2 Polypeptiden mit N-glykosyliertem Asparagin entsprechend Asn546 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, ist und vorzugsweise mit einem Glykan der Strukturen nach Formel 1, 2, 3, 4, 5, 6, 7 oder 8 wie in Definition 4 definiert.
Definition 29. Präparation nach einem der Definitionen 19 bis 28, dadurch gekennzeichnet, dass der Anteil an ACE2 Polypeptiden mit N-glykosyliertem Asparagin entsprechend Asn690 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, ist und vorzugsweise mit einem Glykan der Strukturen nach Formel 1, 2, 3, 4, 5, 6, 7 oder 8 wie in Definition 4 definiert.
Definition 30. Präparation nach einem der Definitionen 19 bis 29, dadurch gekennzeichnet, dass der Anteil an ACE2 Polypeptiden mit O-glykosyliertem Serin entsprechend Ser740 der SEQ ID NO: 1 größer als 60%, vorzugsweise größer als 70%, insbesondere bevorzugt größer als 80%, speziell bevorzugt größer als 90%, am meisten bevorzugt größer als 99%, insbesondere 100%, ist.
Definition 31. ACE2 Polypeptid oder Präparation nach einem der Definitionen 1 bis 30 mit einer katalytischen Aktivität kkat von mindestens 4 /s, vorzugsweise von mindestens 5 /s, insbesondere bevorzugt von mindestens 6 /s, speziell bevorzugt von mindestens 7 /s, am meisten bevorzugt von mindestens 7,6 /s, bezogen auf den Umsatz von AngII (Angiotensin II) zu Ang 1-7 (Angiotensin 1-7).
Definition 32. Verfahren zur Herstellung von rekombinanten ACE2 Polypeptiden oder einer Präparation von rekombinantem ACE2, vorzugsweise nach einem der Definitionen 1 bis 31, umfassend die Schritte der Einbringung eines ACE2 codierenden Polynukleotides, vorzugsweise eines ACE2 ohne Transmembrandomäne codierenden Polynukleotides, in eukaryontischen Zellen, Expression des ACE2 Polypeptids und Sammeln des exprimierten ACE2, insbesondere in dimerer Form, wobei vorzugsweise bei der Expression die Zn²⁺-Konzentration mindestens 1,5 micromolar ist.
Definition 33. Verfahren nach Definition 32, dadurch gekennzeichnet, dass das ACE2 codierende Polynukleotid auf einem Vektor vorgesehen ist.
Definition 34. Verfahren nach Definition 32 oder 33, dadurch gekennzeichnet, dass die ACE2-Expression mit einem Marker, vorzugsweise DHFR, selektiert wird.
Definition 35. Verfahren nach Definition 34, dadurch gekennzeichnet, dass der Marker auf dem Vektor vorliegt.
Definition 36. Verfahren nach Definition 35, dadurch gekennzeichnet, dass der Vektor eine IRES für die exprimierte ACE2 mRNA aufweist.
Definition 37. Verfahren nach einem der Definitionen 32 bis 36, dadurch gekennzeichnet, dass die eukaryontischen Zellen CHO Zellen sind.
Definition 38. Rekombinantes ACE2 Polypeptid oder Präparation von rekombinanten ACE2 Polypeptiden erhältlich durch ein Verfahren der vorhergehenden Definitionen.
Definition 39. Stabil mit einem ACE2 codierenden Polynukleotid transfizierte eukaryontische Zellinie, vorzugsweise CHO Zelllinie, die ACE2 nach einem der Definitionen 1 bis 31 exprimiert.
Definition 40. Zelle nach Definition 39, die eine ACE2-Produktivität von mindestens 10 pg/Zelle/Tag, vorzugsweise mindestens 15 pg/Zelle/Tag, insbesondere bevorzugt von mindestens 20 pg/Zelle/Tag aufweist.
Definition 41. Medikament umfassend ein rekombinantes ACE2 Polypeptid nach einem der Definitionen 1 bis 18, 31 oder 38 oder eine Präparation nach einem der Definitionen 19 bis 31 oder 38.

Die vorliegende Erfindung wird durch die folgenden Figuren und Beispiele weiter illustriert ohne auf diese beschränkt zu sein.

### Figuren:

Fig.1: ACE2 Expressions- und Selektionskassette
Fig.2: ACE2 spezifische Western Blot Analyse der Produktionsklongeschichte
Fig.3: SDS-PAGE Analytik des ACE2 Monomers
Fig.4: Klonauswahl

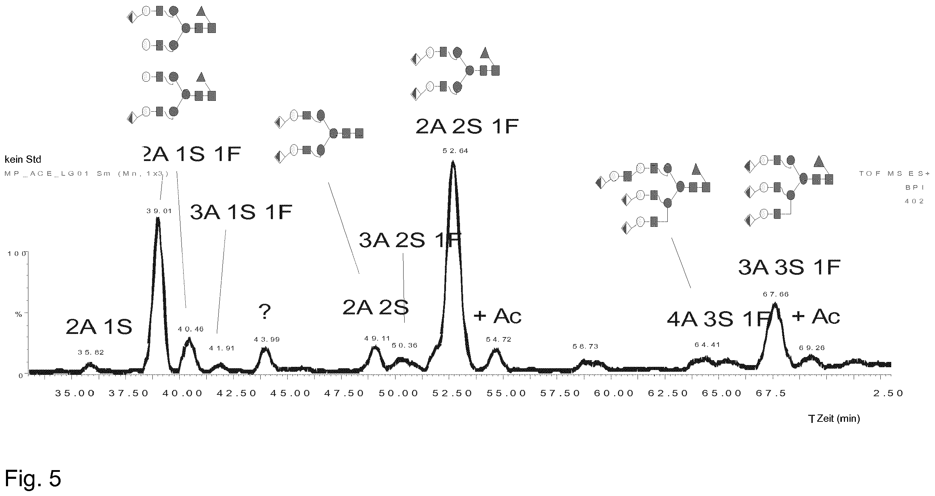

Fig.5: LC-MS Glykosylierungsanalytik
Fig.6: Präparative Größentrennung von ACE2
Fig.7: Pharmakokinetik von ACE2 in 3 Spezies
Fig.8: Kalibrationskurven zur Ang 1-7 und Ang II Quantifizierung. Die Peptide wurden im angeführten Konzentrationsbereich mittels RP-HPLC auf Waters C18 µBondapak RP, 2,1x300mm, 10µm, 125Ǻ Säule aufgetrennt.

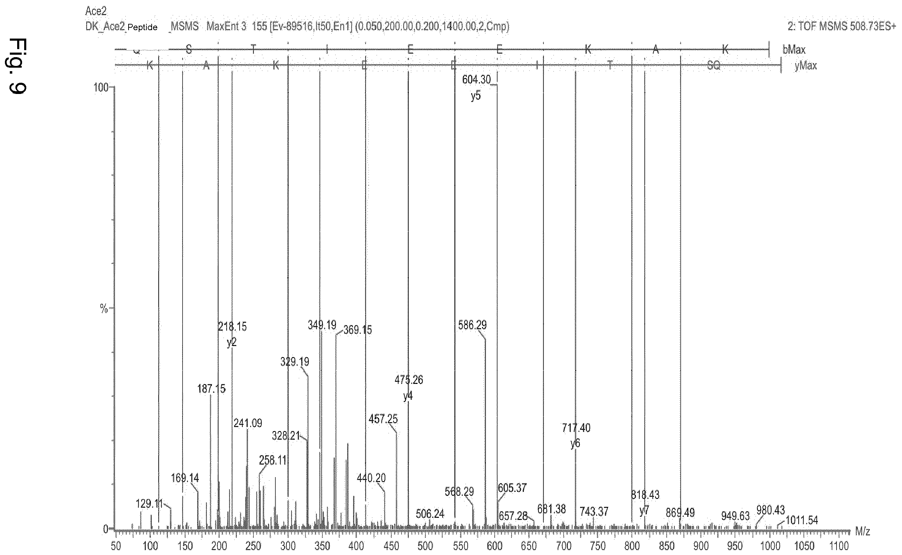

Fig. 9: MS/MS-Spektrum des N-terminalen Peptids. Anmerkung: Q und K haben die gleiche Masse.
Fig. 10: Sequenz von ACE2 und N-Glykosylierungs-Vorhersage
Fig. 11: aufgeschlüsseltes Spektrum für Glykosylierungsstellen 103 (A), Stelle 432 (B), Stelle 546 (C), Stelle 690 (D), Stelle 90 (E).

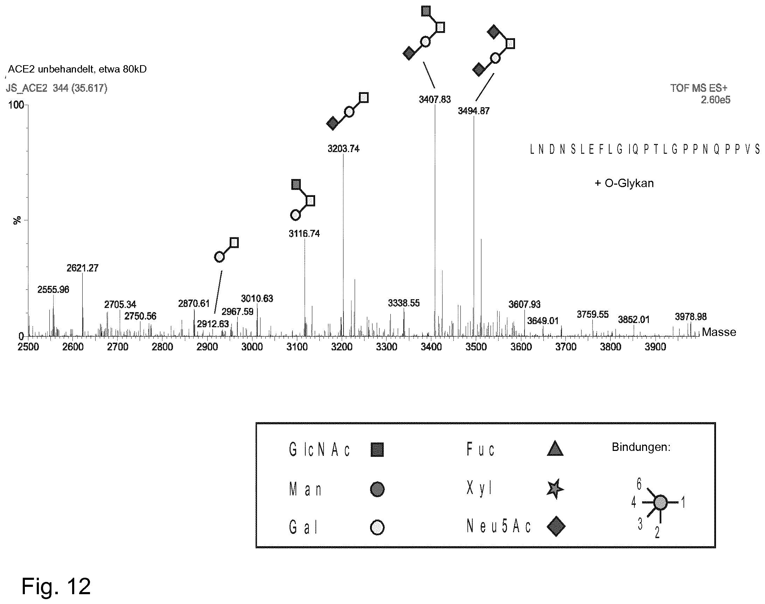

Fig. 12: Spektrum des C-terminalen O-glykosylierten Peptids. Die strukturelle Zuordnung muss als probeweise klassifiziert werden.
Fig. 13: LC-MS freigesetzter Glykane.
Fig. 14: Bestimmung der ACE2 Dimerstruktur mittels nativem PAGE (links, Proteinbanden wurden mittels Silberfärbung visualisiert) und SEC (rechts, Trennung auf einer Zorbax G-450 Säule in Gegenwart von 220 mM Na-Phosphat bei pH 7.0 in 10% Acetonitril, das Chromatogram wurde bei 214 nm aufgenommen.
Fig. 15: Chromatogramm einer Größenausschlusschromatographie des Dimeren ACE2 (Retention 8,55 min, 8,93 min). Standard: Thyroglobulin (670 kDa, 7,43 min), Gamma-globulin (158 kDa), Ovalbumin (43 kDa, 10,08 min), Myoglobulin (17 kDa, 11,08 min), Vitamin B-12 (1,3 kDa, 12,71 min).
Fig. 16: ACE2 spezifische Western Blot Analytik von Zellextrakten aus Cortex (A), Gehirn (B) und einem ACE2-Dimer Expressionsklon (C). In D wurde ein reines ACE2-Dimer aufgetragen.
Fig. 17: Analytisches SEC-HPLC Chromatogram der ACE2 Monomerform. Laufbedingungen: Säule: Zorbax GF250, Puffer: 220 mM Na₂HPO₄ + 10% CH₃CN, pH 8.0 bei 1 ml/min.
Fig. 18: PAGE Analytik von ACE2-Dimer (A) und ACE2 Monomeren (B), Proteine wurden mittels Silberfärbung (a) und ACE2 spezifischem Western-blot (b) nachgewiesen.
Fig. 19: Bestimmung der enzymatischen Aktivität von ACE2 Monomeren im Vergleich zu ACE2-Dimer. Es wurden bei konstanter Anfangskonzentration des fluorenzenzmarkierten Substrates Cumarin-APK-DNP und vier unterschiedliche Enzymkonzentrationen eingesetzt und die jeweiligen Fluoreszenzkurven verglichen.
Fig. 20: ACE2 Serumaktivität gemessen 24 und 48 Stunden nach ACE2-Dimer-Gabe (2,5 mg/kg, blaue Balken) oder ACE2 Monomerform-Gabe (2,5 mg/kg, graue Balken)

### Beispiele:

### Beispiel 1: Expression von hochglykosyliertem ACE-Dimer

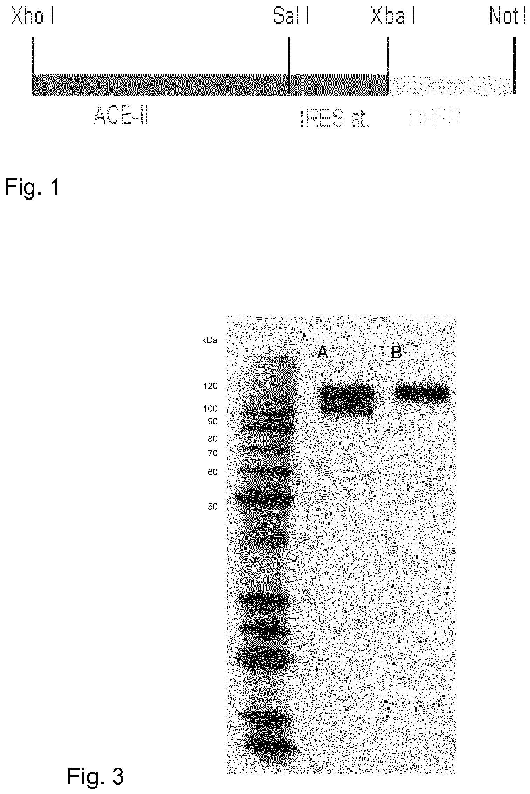

Der lösliche Abschnitt der humanen ACE2 Sequenz (SEQ ID NO:1) wurde in einen Expressionsvektor kloniert, in welchen zuvor der amplifizierbare Selektionsmarker DHFR eingefügt wurde, um zu einer erhöhten Expression des ACE2 Genes zu führen. Hierzu wurde zwischen die für ACE2 und DFHR kodierenden Gene eine attenuierte IRES eingebracht, welche die bi-cistronische Ablesung von ACE2 und DHFR auf derselben mRNA ermöglicht. Die ACE2 Expressions- und Selektionskassette ist in Figur 1 graphisch dargestellt. Nachdem beide Proteine unter Kontrolle desselben Promotors exprimiert werden, kann über DHFR Selektion unter Verwendung des Antagonisten MTX zielstrebig die ACE2 Expression gesteigert werden. Durch diese Strategie ist es möglich, besonders stabile Expressionszelllinien zu erhalten, die hohe Ausbeuten eines Produktes konstanter Qualität liefern. Dies ermöglicht es auch in Zelllinien vernünftige Produkttiter zu erzielen, die möglicherweise für die rekombinante Expression eines bestimmten Targetproteins minder geeignet sind.

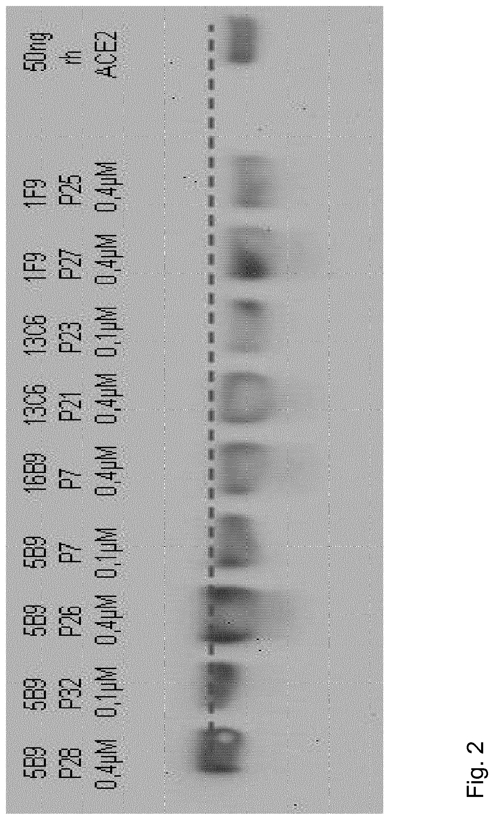

Dieser Vektor wurde in CHOdhfr- Zellen transfiziert und unter kontinuierlich gesteigertem MTX Druck wurde die Kopienanzahl der ACE2 Gene amplifiziert. Über mehrere Selektions- und Subklonierungsrunden wurden die besten Produzenten mittels intrazellulärer FACS Analytik und proteinchemischer sowie enzymatischer Analysen auf optimale Produkteigenschaften selektiert: Es wurden für die Auswahl des bestgeeigneten Klons vor allem die spezifische enzymatische Aktivität, welche mit 3 verschiedenen Substraten gemessen wurde, die Produkthomogenität, die zelluläre Produktivität, jedoch auch die Zuckerkomplexizität in Betracht gezogen. In Fig. 2 ist eine rekapitulative Western Blot Analyse der sukzessiven Kulturüberstände einzelner Klone dargestellt, die zur Etablierung der Produktionszelllinie verwendet wurden. Die Produkteigenschaften der einzelnen Klone unterscheiden sich insofern, dass der Zuckeranteil der Expressionsprodukte von rechts nach links zunimmt, was durch eine deutliche Massenzunahme erkennbar ist. Dies wurde durch spezifische Selektion hochglykosylierender Klone erreicht. Letztendlich wurde ein Klon (Klon 5B9), welcher das Expressionsprodukt mit dem höchstem Molekulargewicht aufwies, zur Etablierung der Produktionszelllinie (1B4) verwendet.

Es wurde entschieden, 6 Klone weiterzuführen, die enzymatisch hoch aktives und komplex N-glykosyliertes ACE2 exprimierten. Während lösliches ACE2 ein Molekulargewicht von 83 kDa aufweist, werden Klone ausgewählt, welche im denaturierenden SDS-PAGE, bedingt durch deren Zuckerstruktur, im Bereich von bis zu 120 kDa erschienen. Fig. 3 zeigt ein durch den vorliegenden Produktionsprozess hergestelltes ACE2 (Lane B) im Vergleich zu einem in NS0 hergestellten ACE2 Standard (Lane A). Während das erfindungsgemäße ACE2 Polypeptid - hier als Monomer analysiert - homogen, hoch glykosyliert ist und daher als einzelne Bande bei ca. 120 kDa erscheint weist das Referenzmaterial Banden von 83 kDa bis zu 120 kDa auf, was eine sehr heterogene Glykosylierung andeutet.

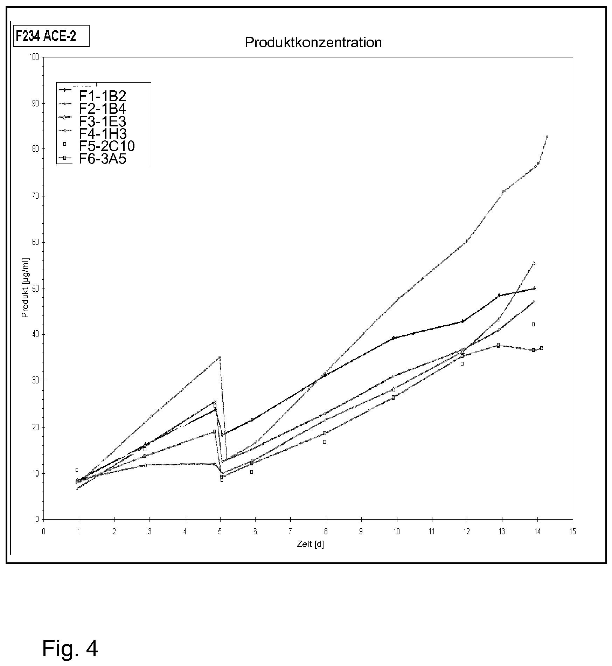

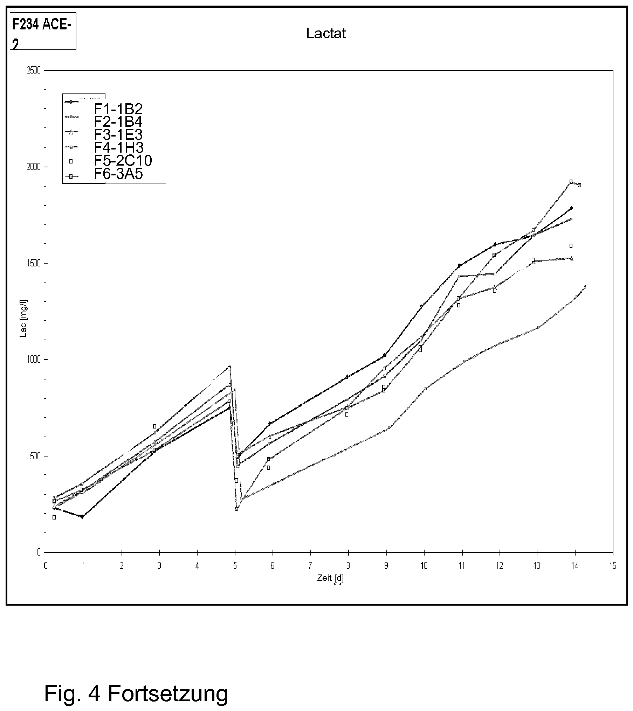

Die präliminären Klone wurden im Weiteren auf proteinfreies Wachstumsmedium (Polymun) umgestellt. Dieses käufliche Medium ist chemisch definiert, Serum-frei, frei von tierischen Proteinen und auf die rekombinante Expression von Glykoproteinen in CHO optimiert. Die Fermentation wurde bei 2,5-3,5 µM Zn²⁺ durchgeführt. Alle 6 Klone wurden in Kultur gehalten und auf deren Produktionsprozesstauglichkeit überprüft. Im Speziellen wurden Wachstumsraten aufgezeichnet sowie die Überstände auf Produktverlauf und Metaboliten untersucht (Fig. 4). Wiederum wurden die Expressionsprodukte sowie die Klone genau analysiert. Alle Klone exprimierten hochaktives ACE2 und wiesen Produktivitäten um 20-30 pg/Zelle/Tag auf. Weiters wurden ebenfalls die Zuckerstrukturen und deren Heterogenität analysiert. Letztendlich wurde Klon 1B4 ausgewählt. Er wies über den gesamten Produktionsverlauf eine homogene Zuckerstruktur auf. Es waren alle 7 N-Glykosylierungsstellen prozessiert, wobei diese mindestens eine bi-, manche jedoch auch eine triantennäre komplexe Glykosylierung mit terminalen Sialinsäuren aufwiesen.

Auf der Basis dieses Klones wurde eine Mastercellbank hergestellt und getestet, sowie ein GMP-tauglicher Reinigungs- und in weiterer Folge ein GMP Produktionsprozess aufgebaut.

SEQ ID NO: 1 (ACE2 Proteinsequenz, die autologe Signalsequenz (unterstrichen) wird durch die Wirtszelle für die Ausschleusung abgespaltet):

MSSSSWLLLSLVAVTAA
QSTIEEQAKTFLDKFNHEAEDLFYQSSLASWNYNTNITEENVQNMNNAGDKWSAFLKEQSTLAQMYP
LQEIQNLTVKLQLQALQQNGSSVLSEDKSKRLNTILNTMSTIYSTGKVCNPDNPQECLLLEPGLNEIMANSLDYN
ERLWAWESWRSEVGKQLRPLYEEYVVLKNEMARANHYEDYGDYWRGDYEVNGVDGYDYSRGQLIEDVEHTFE
EIKPLYEHLHAYVRAKLMNAYPSYISPIGCLPAHLLGDMWGRFWTNLYSLTVPFGQKPNIDVTDAMVDQAWDA
QRIFKEAEKFFVSVGLPNMTQGFWENSMLTDPGNVQKAVCHPTAWDLGKGDFRILMCTKVTMDDFLTAHHEM
GHIQYDMAYAAQPFLLRNGANEGFHEAVGEIMSLSAATPKHLKSIGLLSPDFQEDNETEINFLLKQALTIVGTLPF

TYMLEKWRWMVFKGEIPKDQWMKKWWEMKREIVGVVEPVPHDETYCDPASLFHVSNDYSFIRYYTRTLYQFQ
FQEALCQAAKHEGPLHKCDISNSTEAGQKLFNMLRLGKSEPWTLALENVVGAKNMNVRPLLNYFEPLFTWLKDQ
NKNSFVGWSTDWSPYADQSIKVRISLKSALGDKAYEWNDNEMYLFRSSVAYAMRQYFLKVKNQMILFGEEDVR
VANLKPRISFNFFVTAPKNVSDIIPRTEVEKAIRMSRSRINDAFRLNDNSLEFLGIQPTLGPPNQPPVS

Bedingt durch die Dimerisierung von ACE2 sind sämtliche hydrophobe Proteineinheiten in das Innere des Komplexes gerichtet, wobei die geladenen Reste, wie N-gebundenen Zuckerketten nach außen ragen und die Struktur im ebenfalls geladenen physiologischen Milieu solvatisieren. Diese Dimerisierung durch Expression eines vollständig N-glykosylierten ACE2 wurde in Gegenwart von Zn²⁺ festgestellt. Der Dimer-Komplex besteht hierbei aus 2 identischen Untereinheiten, die elektrostatisch aneinander gebunden sind und sich auch nicht mehr in physiologischen Lösungen trennen. Es kommt hierbei zur Sekretion eines Glykoproteins mit jeweils 14 stark geladenen Sialinsäurestrukturen auf jedem ACE2 Molekül sowie 28 Sialinsäurestrukturen im Dimer. Jeweils 2 Zn²⁺ Ionen werden in den Komplex eingebaut und stabilisieren dessen Struktur. Die starke Ladung der Zuckerketten solvatisiert das Molekül in physiologischen wäßrigen Lösungen und zwingt die zugehörigen geladenen Proteindomänen nach außen. Der Produktionsprozeß wurde so aufgebaut, dass im Endprodukt ausschließlich ACE2 Dimere vorkommen.

Dies wird dadurch ermöglicht, dass bei Generierung des ACE2 genügend Zn²⁺-Ionen vorhanden sind (vorzugsweise kommen 1,5-5 micromolar Zn²⁺ zur Anwendung, insbesondere kann die Fermentation bei 2,5-3,5 uM Zn²⁺ durchgeführt werden) und anschließend die weiteren Behandlungsschritte in Anwesenheit von Zn²⁺-Ionen vorgenommen werden.

Die Aufreinigung erfolgte unter Verwendung eines Anionentauschschrittes, einer Ammoniumsulfatpräzipitation, eines HIC-Schrittes, eines Kationentauscherschrittes sowie eines weiteren hochauflösenden Anionentauscherschrittes. Alle Medien im Prozeß waren phosphatgepuffert und enthielten Natriumchlorid. Der finale Probenpuffer ist eine physiologische Glycinlösung zur Injektion.

### Beispiel 2: Produkteigenschaften

Komplexes, hochglykosyliertes ACE2 Monomer bzw. Monomereinheiten des Dimers weisen aufgrund der kovalent gebundenen Zuckerstrukturen ein deutlich höheres Molekulargewicht auf als es der Aminosäuresequenz entspricht. So wurde mittels Peptide-map eine Molmasse von 102 kDa gemessen, während die von nicht-glykosyliertem ACE2 nur 83 kDa beträgt. Der Zuckeranteil liegt dementsprechend bei 23% und ist für die im Weiteren beschriebenen Produkteigenschaften von essentieller Bedeutung. Aufgrund starker Hydratisierung erscheint die Monomereinheit in der SDS-PAGE bei ca. 120 kDa (Fig. 3) im Vergleich zu den Referenzstandards.

Nicht das natürliche, membrangebundene ACE2 wurde exprimiert, sondern ausschließlich der lösliche Anteil von ACE2. Aufgrund der fehlenden Membrandomäne wird es vollständiger glykosyliert.

Aufgrund der komplexen, hoch-sialylierten und somit stark negativ geladenen Zuckerstrukturen besitzt ACE2 eine deutlich höhere Löslichkeit im Vergleich zu nicht glykosyliertem oder unvollständig glykosyliertem ACE2. Somit können physiologisch gepufferte Proteinformulierungen von bis zu 15 mg/ml problemlos hergestellt werden.

Das Produkt verhält sich ferner auch stabil in Lösung und verliert über einen längeren Lagerungszeitraum weder Aktivität, noch neigt es zum Abbau oder zur Aggregation, abgesehen von der stabilen Dimerisierung. Deglykosyliertes ACE2 hingegen fällt bereits im geringen Konzentrationsbreich aus. Dies konnte in einem präparativen Deglykosylierungsansatz mittels PNGaseF gezeigt werden. Der für ACE2 berechnete, theoretische Stabilitätsindex beträgt 41,2 und klassifiziert das Protein als instabil, wobei bei dieser Berechnung die Zuckerstrukturen außer Acht gelassen wurden. Nachdem sich die Formulierungen jedoch monatelang in Lösung stabil verhalten, ist dies offensichtlich auf den hohen Zuckeranteil zurückzuführen. Die bessere Solvatisierung von ACE2 führt weiters dazu, dass diese Formulierung ausschließlich aus ACE2 Dimeren (bzw. nach künstlicher Trennung zum Monomer ausschließlich aus Monomeren) besteht, während nicht glykosyliertes ACE2 zur Multimerisierung und zur Aggregation neigt.

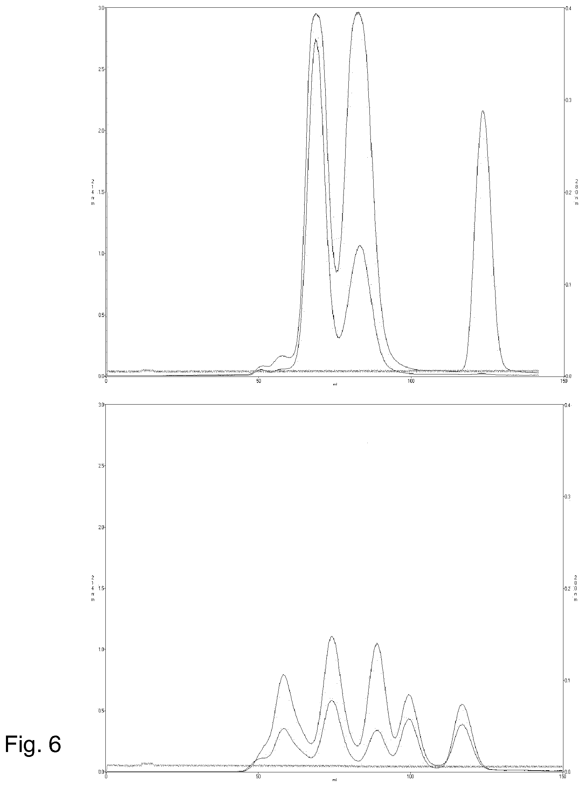

Aufgrund des hohen Anteils geladener Zuckerreste nimmt weiters der hydrodynamische Durchmesser, die Solvatisierungshülle der vorliegenden ACE2 Präparation stark zu. Dieser Umstand kann zur präparativen Aufreinigung von ACE2 über Größentrennungssäulen genutzt werden, wo das hier ausschließlich als Dimer vorliegende Protein bei einem scheinbaren Molekulargewicht von 250 kDa im Vergleich zu den berechneten 83 kDa erscheint. Ein Chromatogramm einer präparativen ACE2 Reinigung ist in Fig. 6 wiedergegeben. ACE2 (erste Abbildung) eluiert mit einer Retentionszeit von 69 Minuten. Dies entspricht einem scheinbaren Molekulargewicht zwischen dem von Thyroglobulin (erste Abbildung bei 58 Minuten, 670 kDa) und Gamma-Globulin (erste Abbildung bei 74 Minuten, 158 kDa). In diesem Bereich hoher Molekulargewichte kommen in Kulturüberständen so gut wie keine Kontaminationen vor, sodass hochreines Produkt in einem sehr einfachen Trennungsschritt abgetrennt werden kann.

### Beispiel 3: Pharmakologische Produkteigenschaften

Die erfindungsgemäße ACE2 Präparation liegt als stabile, hochreine und konzentrierte Proteinlösung in physiologischer Pufferung vor und kann ohne weitere Stabilisierung gelagert und verabreicht werden. Z.B. kommt ein Phosphatpuffer zum Einsatz.

ACE2 ist als Monomer oder Dimer stabil in Lösung und zeigt aufgrund des hohen Zuckeranteils keine Aggregation. Ferner weist die ACE2 Präparation die volle enzymatische Aktivität auf.

ACE2 kann aufgrund seiner Löslichkeit als Bolus i.v. verabreicht werden. Die Bioverfügbarkeit ist aus denselben Gründen systemisch sofort nach Gabe gewährleistet.

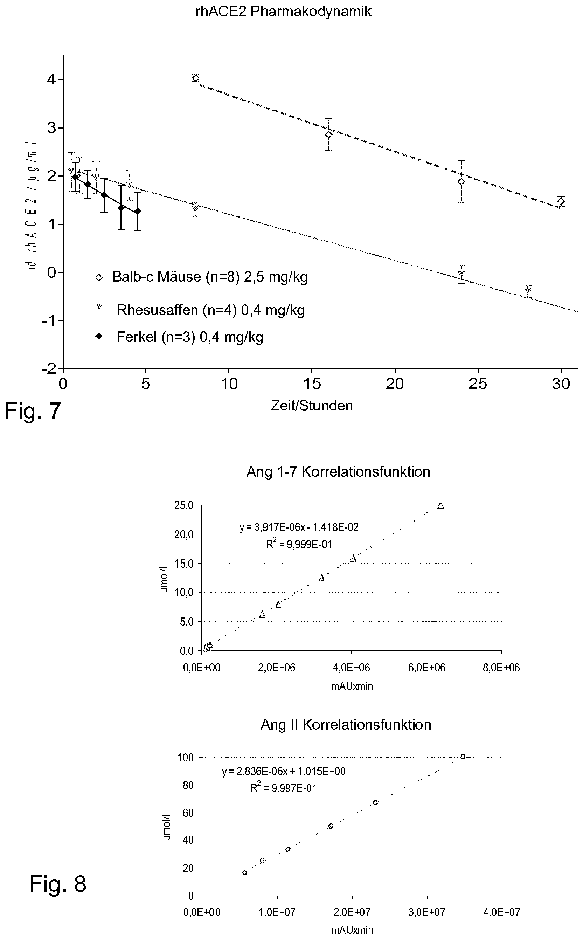

Aufgrund des hohen, stark verzweigten und komplexen Zuckeranteils wird ACE2 nur langsam abgebaut. Es resultiert daraus eine lange Halbwertszeit von mindestens 10.5 Stunden, welche in diversen Spezies, vor allem auch in Rhesus Makaken gemessen wurde. Fig. 7 stellt die gemessene Halbwertszeit von i.v. gegebenem ACE2 in 3 Spezies dar.

Der hohe Sialinsäureanteil bedingt weiters, dass gegen ACE2 keine neutralisierende Immunantwort aufgebaut wird. Diese wäre nicht nur für die exogene Gabe von ACE2 kontraproduktiv, sondern könnte auch autologes, zellständiges ACE2 neutralisieren.

Die beschriebene ACE2 Formulierung mitsamt sämtlicher einhergehender Produkteigenschaften ermöglicht daher erst eine effiziente Therapie mit rhACE2.

### Beispiel 4: Bestimmung der spezifischen ACE2 Aktivität

Die spezifische Aktivität von ACE2 Präparationen wurde durch Messung des Umsatzes von Ang II (Asp-Arg-Val-Tyr-Ile-His-Pro-Phe) bestimmt. Sämtliche Messungen wurden als Dreifachbestimmungen in einem Ansatzvolumen von 100 µl durchgeführt. Die enzymatische Reaktion wurde durch Zugabe von 250 ng/ml ACE2 zu einer 80 µM Ang II Lösung in 50 mM MES, 300 mM NaCl, 10µM ZnCl₂ und 0,01% Brij-30 bei pH 6,5 gestartet. Die Proben wurden sorgfältig gemischt und für genau 18 Minuten bei 37°C inkubiert. Die enzymatische Reaktion wurde durch Zugabe von 100 mM EDTA gestoppt. Zur Analyse wurden die Lösungen mittels RP-HPLC (Waters C18 µBondapak, 2,1x300mm, 10µm, 125Ǻ) unter Verwendung eines linearen Gradienten von 10 bis 60% CH₃CN in 0,08% H₃PO₄ über 20 Minuten bei einer Flussrate von 1 ml/min aufgetrennt. Weiters wurden sowohl Ang II als auch Ang 1-7 Peaks in den Chromatogrammen erkannt und integriert. Die Peptidkonzentrationen wurden anhand der jeweiligen in Fig. 8 dargestellten Kalibrationskurven ermittelt. Weiters wurde der enzymatische Umsatz sowie die spezifische Enzymaktivität bestimmt.

Die Aktivität des ACE2 Produktes wurde wie zuvor beschrieben bestimmt. In Tabelle 1 sind die jeweiligen Resultate der Peakintegration sowie die berechneten Enzymdaten dargestellt.

**Tabelle 1: Enzymdaten und Reaktionsbedingungen. Es sind die Mittelwerte von Dreifachbestimmungen angegeben.**

| Enzymatische Reaktivität | | | | | |
|---|---|---|---|---|---|
| **Peak Fläche** mAU.min | **Umsatz** µmol.ml⁻¹ | **Reaktionszeit** min | **ACE2 Konz.** ng.ml⁻¹ | **kkat** s⁻¹ | **Spezif. Aktivität** µmol.mg⁻¹.min⁻¹ |
| **Ang II** | | | | | |
| 17149890 | 62,1 | 18 | 250 | 8,0±0,3 | 4,7±0,2 5 |

| **Ang 1-7** | | | | | |
|---|---|---|---|---|---|
| 4720141 | 23,1 | 18 | 250 | 8,8±0,2 | 5,2±0,1 |

Die ACE2 Präparation weist eine katalytische Aktivität kkat von 8,0±0,3 /s gemessen anhand des Ang II Umsatzes und 8,8±0,2 /s in Bezug auf den Ang 1-7 Umsatz auf. Beide Werte stimmen gut überein und sind deutlich höher als die Angaben von Vickers et al., (J Biol Chem. 277 (17) (2002): 14838-43), wo eine katalytische ACE2 Aktivität von 3,5 /s veröffentlicht wurde. Die Reaktionbedingungen waren identisch. Die Ursache der 240% höheren Aktivität unserer Präparation dürften post-translationale Modifikationen und hier vor allem die N-Glykosylierung sein, die in dem Material, das Vickers verwendet hat, deutlich geringer ausgeprägt war. Dieses Material wurde in Insektenzellen exprimiert und wies zwar dieselbe Aminosäuresequenz auf, war jedoch zu einem deutlich geringeren Anteil und Verzweigungsgrad glykosyliert. Es wurde weiters auch eine im Handel erhältliche ACE2 Präparation von R&D systems (cat. 933-ZN), die ebenfalls eine deutlich geringere Aktivität kkat von 2,0±0,1 /s aufwies, untersucht. Eine wesentliche Eigenschaft der erfindungsgemäßen Präparation ist die besonders hohe enzymatische Aktivität, die vor allem durch post-translationale Modifikationen ermöglicht wird.

### Beispiel 5: Glycoproteomische Analyse von rekombinantem ACE2

Die Probe von gereinigtem, CHO-exprimiertem ACE2 wurde auf zweierlei Arten analysiert:
Erstens wurden tryptische Peptide nach SDS-PAGE und S-Alkylierung erzeugt und mittels LC-ESI-MS (und MS-MS) analysiert. Das N-terminale Peptid und mehrere interne Peptide wurden gefunden. Fünf der sieben potentiell N-glykosylierten Stellen wurden in glykosylierter Form mit Glykanstrukturen gefunden, die hauptsächlich di-antennäre Glykane mit Fucose und verschiedene Mengen an Sialinsäure enthielten. Das C-terminale Peptid scheint O-glykosyliert zu sein.

Zweitens wurden freie, reduzierte N-Glykane mittels Carbon-LC-ESI-MS analysiert. Für das di-antennäre, di-sialysierte Glykan mit Fucose konnte gezeigt werden, dass die Fucose mit dem Kern a 1,6-verbunden ist und die Sialinsäure a 2,3-verbunden ist. Zusätzlich zu mono- oder disialysierten, di-antennären Glykanen wurde eine beträchtliche Menge an tri-antennärem Oligosaccharid gefunden. Eine grobe Schätzung der Masse des glykosylierten ACE2 ergibt 101-102 kDa, d.h. bestehend aus etwa 17% Kohlehydrat.

### Proteolytischer Verdau von mittels SDS-PAGE getrennten hBChE

Aliquots von ACE2 wurden einer SDS-PAGE unterzogen, entfärbt, carbamidomethyliert, mit Trypsin von Sequenzier-Qualität verdaut und aus Gel-Stücken, wie beschrieben, extrahiert. Die Extrakte wurden in einem Speed Vac-Konzentrator zur Trockene gebracht und mit Wasser, das 0,1% Ameisensäure enthielt, vor der nachfolgenden LC-MS-Analyse rekonstituiert. Für die Oligosaccharid-Analyse wurden die Peptide mit PNGase F verdaut, und die Glykane wurden unter Verwendung von C18 SPE-Spitzen gereinigt.

### MS-Analyse tryptischer Peptide und Glykopeptide

Die massenspektrometrische Analyse wurde auf Q-TOF Ultima Global (Waters Micromass), ausgerüstet mit einer Standard-Elektrosprüheinheit, einem Cap-LC-System (Waters Micromass) und einem 10-Port-Lösungsmittel-Wechsel-Modul (Rheodyne), wie kürzlich beschrieben [Kolarich 2006], durchgeführt. Die Proben wurden zuerst auf einer Aquasil C18-Vorsäule (30 x 0,32 mm, Thermo Electron) unter Verwendung von Wasser als Lösungsmittel eingefangen. Die Analyse-Säule, eine Biobasic C18-Säule (100 x 0,18 mm, Thermo Electron) wurde vor dem Lösungsmittel-Wechsel auf 5% Acetonitril gehalten und danach wurde ein linearer Gradient von 5 bis 50% Acetonitril mit einer Flussrate von 2 µl/min angelegt. Alle Elutionsmittel enthielten 0,1% Ameisensäure.

Die Proben wurden auf MS- und auch auf MS-MS-Art analysiert. Die Datenanalyse erfolgte mit MassLynx 4.0 SP4 software (Waters Micromass).

### Analyse freier N-Glykane von ACE2

Borhydrid-reduzierte Glykane wurden auf einer porösen graphitischen Kohlenstoff-Säule getrennt und mittels Massenspektrometrie nachgewiesen.

Es wurde versucht, den N-Terminus und den C-Terminus zu finden. Das Protein enthält

### Beispiel 6: Glykosylierungsanalytik

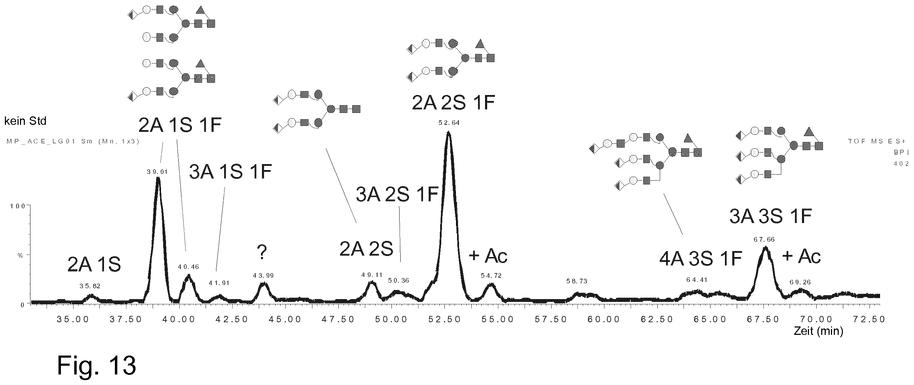

Die Proteinsequenz von ACE2, dessen Molekulargewicht, die Position sämtlicher Glykosylierungsstellen sowie die Struktur der gebundenen Zucker wurden hierin bestimmt. Die Probe wurde mittels tryptischem Verdau, S-Alkylierung und LC-ESI-MS analysiert.
• Die ermittelte Proteinmasse des glykosylierten Produktes beträgt 102 kDa, wobei der Zuckeranteil 23% der Gesamtmasse entspricht.
▪ Der N-terminus sowie die Anwesenheit sämtlicher sequenzinternen Peptide wurden hierbei bestätigt (siehe Sequenzinformation).
▪ Alle 7 postulierten N-Glykosylierungsstellen (Positionen 53, 90, 103, 322, 432, 546 und 690) enthielten tatsächlich di-antennäre, komplexe sialinsäurehältige fukosylierte Zuckerstrukturen (Fig. 13)
▪ Das C-terminale Peptid war O-glykosyliert.

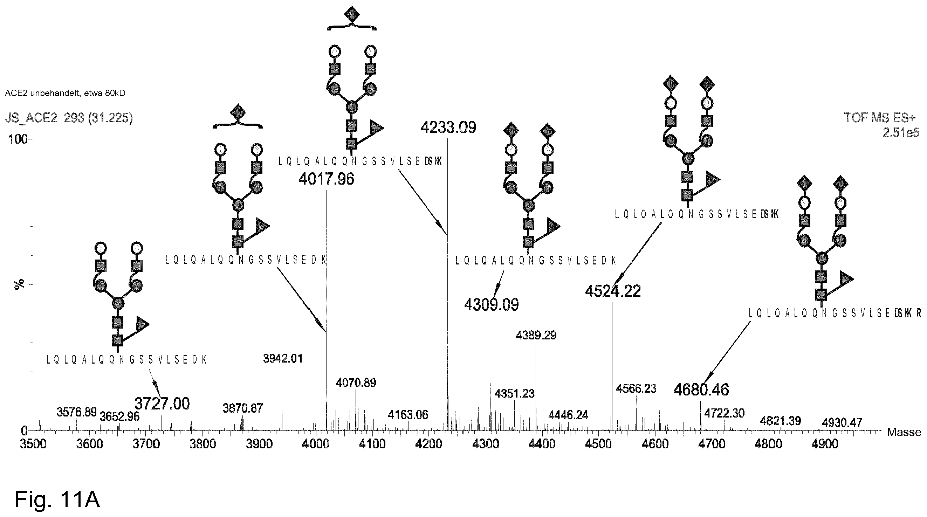

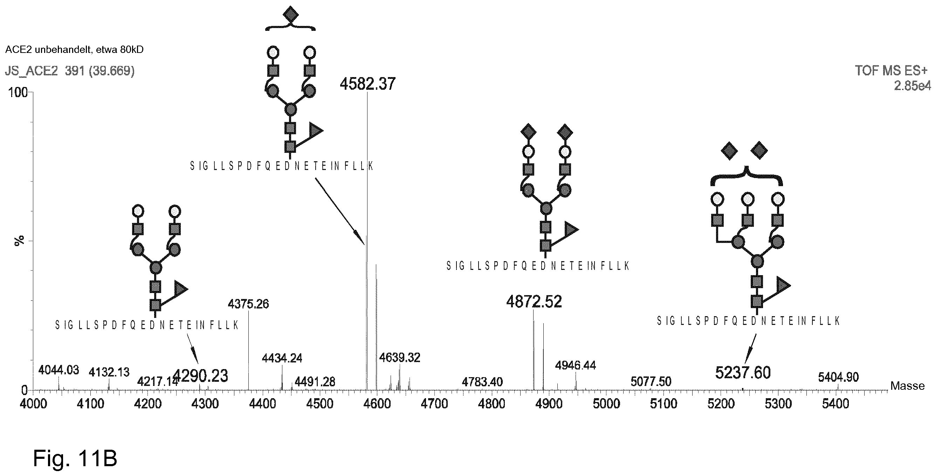

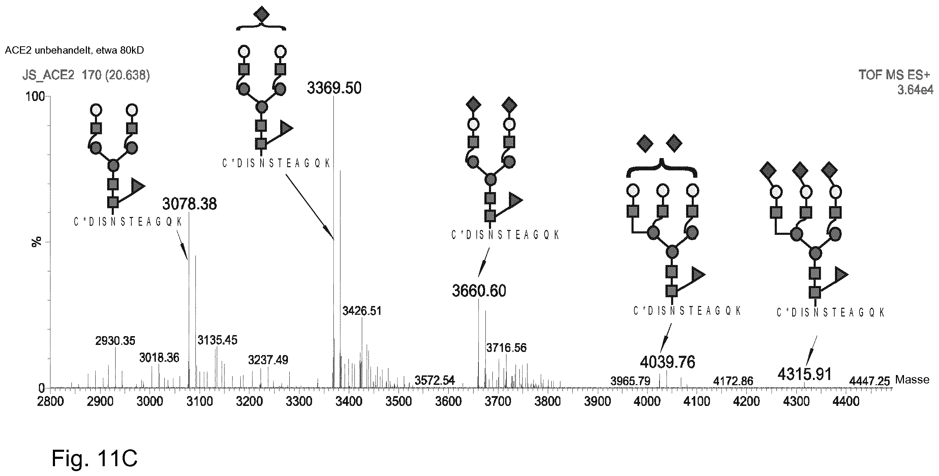

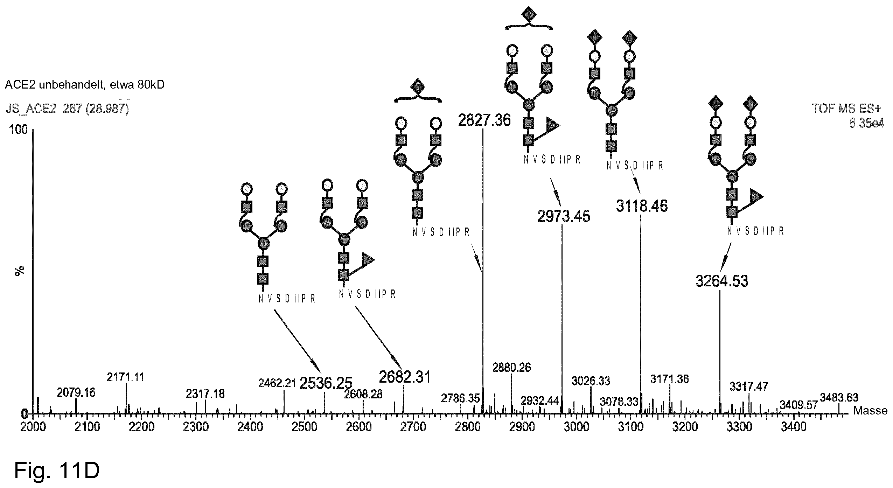

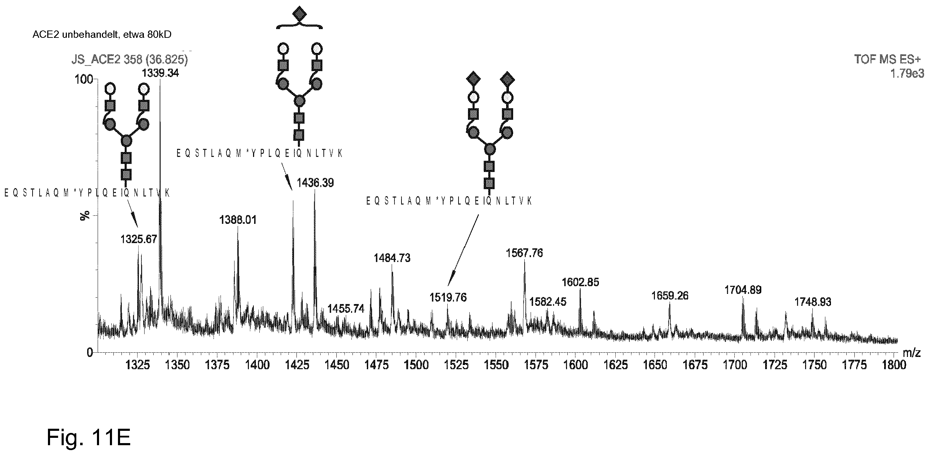

Die Struktur dieser Zuckerreste wurde durch carbon-LC-ESI-MS nach deren Abspaltung und Reduktion bestimmt. Sämtliche di-antennären Stukturen enthielten jeweils zwei a2,3-gebundene Sialinsäuren und eine a1,6-gebundene Fukose. Geringe Mengen triantennärer Strukturen wurden ebenfalls gefunden (Fig. 11).

Die angewendete Selektionstrategie ermöglichte es, zu einem vollständig glykosylierten und sialinsäurehältigen Expressionsprodukt zu kommen.

### Methodik:

### Probenvorbereitung

ACE2 wurde mittels SDS-PAGE aufgetrennt, entfärbt, alkyliert und mittels Trypsin verdaut. (Kolarich et al., Proteomics 6 (2006): 3369-3380). Gelextrakte wurden getrocknet und vor der LC-MS Analytik in 0,1% Ameisensäure gelöst. Für die Zuckeranalytik wurden die Peptide mittels PNGase-F verdaut und über C18 SPE Säulchen gereinigt

### MS-Analytik

Alle Massenspektroskopischen Analysen wurden mittels Q-TOF Ultima Global (Waters Micromass) mit einer Standard-electrospray Einheit und einem Cap-LC system (Waters Micromass) durchgeführt (Kolarich 2006).

Die Proben wurden auf einer Aquasil C18 Vorsäule (30 x 0.32 mm, Thermo Electron) in Wasser angereichert. Als Trennsäule wurde eine C18 Säule (100 x 0.18 mm, Thermo Electron) unter Verwendung eines Acetonitrilgradienten eingesetzt. Die Proben wurden im MS und im MSMS Modus vermessen.

### Analytik der freien N-Glykane

Mittels Borohydrid reduzierte Glykane wurden auf einer porösen Graphit Säule getrennt und über ihre Masse charakterisiert.

### Beispiel 7: Dimerisierung von rhACE2

Nach Beispiel 1 hergestellte ACE2 wird als Dimer erhalten und in diesem Beispiel als solches analysiert, ohne die Dimere zu trennen. Durch native Behandlung bleibt das Dimer erhalten im Gegensatz zu denaturierenden Analysen (Fig. 3). Bedingt durch die Dimerisierung von ACE2 sind sämtliche hydrophobe Proteineinheiten in das Innere des Komplexes gerichtet, wobei die geladenen Reste, wie N-gebundenen Zuckerketten nach außen ragen und die Struktur im ebenfalls geladenen physiologischen Milieu solvatisieren. Diese Dimerisierung durch Expression eines vollständig N-Glykosylierten ACE2 wurde in Gegenwart von Zn²⁺ festgestellt. Der Dimer-Komplex besteht hierbei aus 2 identischen Untereinheiten, die elektrostatisch aneinander gebunden sind und sich auch nicht mehr in physiologischen Lösungen trennen. Es kommt hierbei zur Sekretion eines Glykoproteins mit jeweils 14 stark geladenen Sialinsäurestrukturen auf jedem ACE2 Molekül sowie 28 Sialinsäurestrukturen im Dimer. Jeweils 2 Zn²⁺ Atome werden in den Komplex eingebaut und stabilisieren dessen Struktur. Die starke Ladung der Zuckerketten solvatisiert das Molekül in physiologischen wäßrigen Lösungen und zwingt die zugehörigen geladenen Proteindomänen nach außen. Der Produktionsprozeß wurde so aufgebaut, dass im Endprodukt ausschließlich ACE2 Dimere vorkommen.

Dies wird dadurch ermöglicht, dass bei Generierung des rACE2 genügend Zn²⁺-Ionen vorhanden sind (vorzugsweise kommen 1,5-5 micromolar Zn²⁺ zur Anwendung, insbesondere kann die Fermentation bei 2,5-3,5 uM Zn²⁺ durchgeführt werden) und anschließend die weiteren Behandlungsschritte in Anwesenheit von Zn²⁺-Ionen vorgenommen werden.

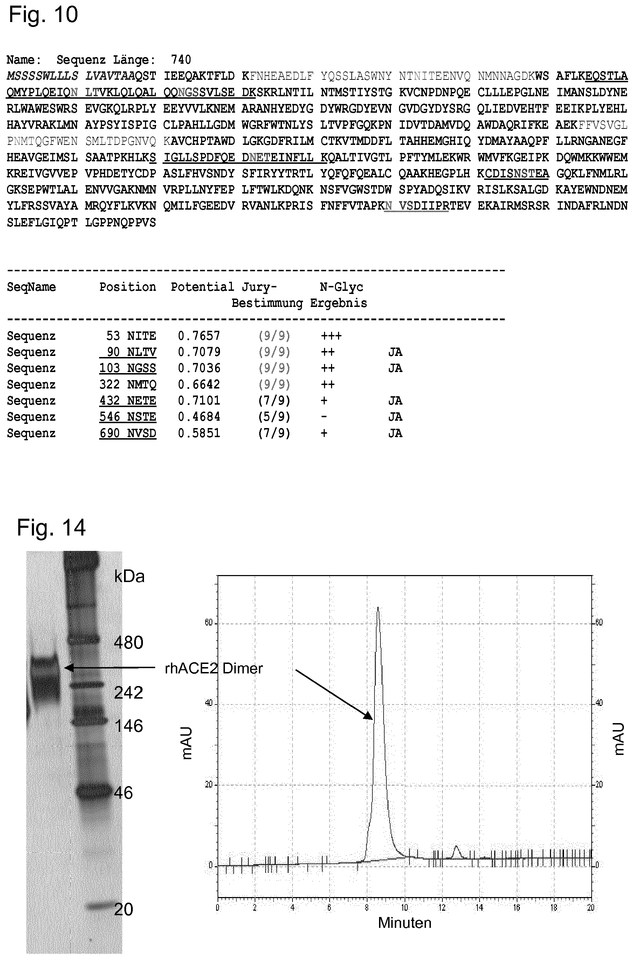

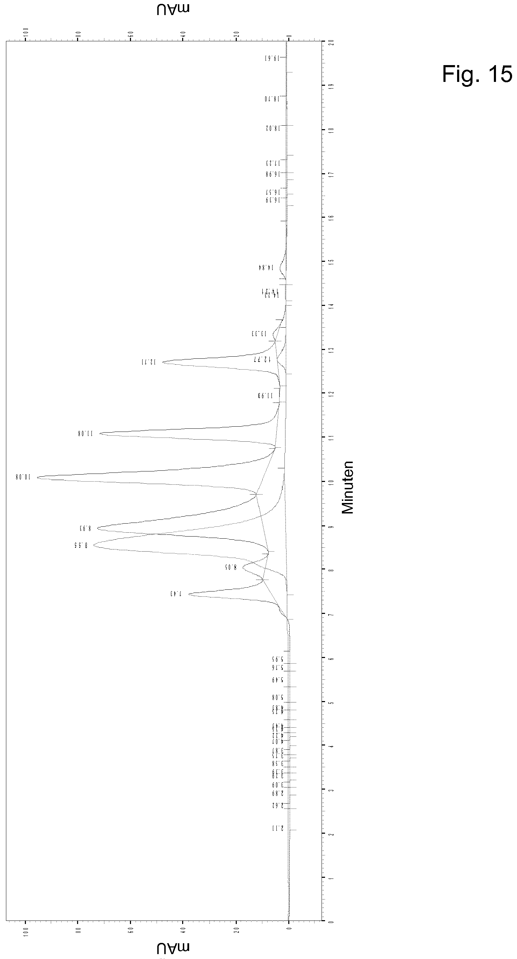

In Figur 14 und 15 wurde die Dimerisierung des ACE2 Komplexes mit unterschiedlichen Methoden nachgewiesen. In der nativen Poly-Acrylamid-Gel-Elektrophorese wurde das Protein nach Silberfärbung als einzelne Bande einer Größe von ca. 250 kDa nachgewiesen. Mittels Größentrennungschromatographie auf einer Zorbax G-450 Säule in Gegenwart von 10% Acetonitril in 220 mM Na-Phosphatpuffer bei pH 7,0 erscheint das Produkt ebenfalls als einzelner Peak bei einer einem Molekulargewicht von ca. 250 kDa entsprechenden Retentionszeit. Bemerkenswert ist in beiden Fällen, dass ausschließlich dimerisiertes Protein nachgewiesen werden kann. Weder Monomerstrukturen, noch höhermolekulare Aggregate konnten nachgewiesen werden.

### Beispiel 8: ACE2 Dimere - Unterschiede zu membranständigem ACE2

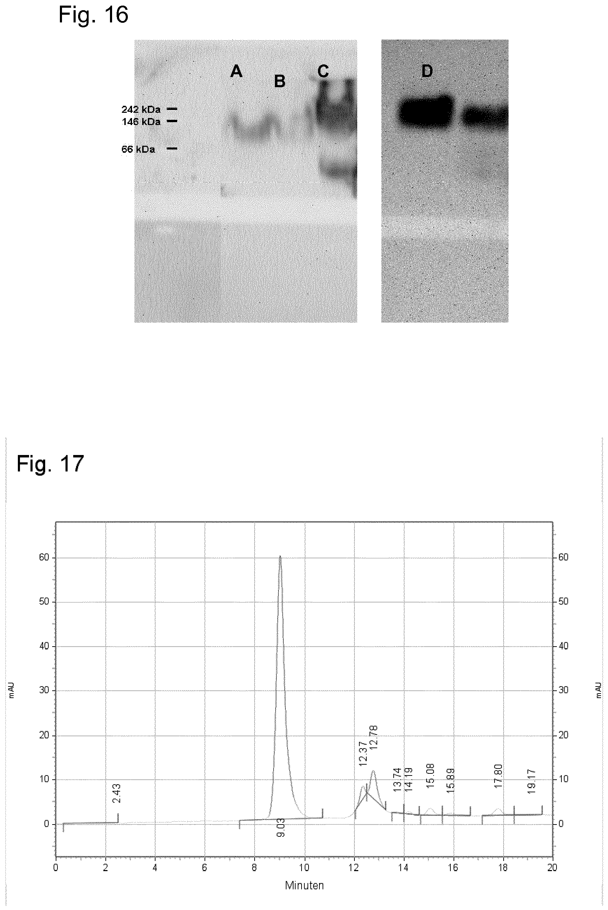

ACE2 wird als Transmembranprotein in allen höheren Spezies vorwiegend auf Nieren-, Herz-, Lungen- und Leberzellen als welsentliches Enzym des Renin Angiotensin Systems exprimiert. Membranverankertes ACE2 umgibt sich naturgemäß in der membranären Lipiddoppelschicht mit anderen Membranproteinen, welche ACE2 in einer aktiven Konformation stabilisieren und ebenfalls die extrazelluläre Domäne von ACE2 vor proteolytischem Abbau schützen. Um die pharmakologischen Eigenschaften von löslichem ACE2 und im Speziellen die Aktivität und Stabilität des löslichen Proteins zu erhöhen, wurde eine Expressions- und Produktionsstrategie gewählt, die ausschließlich zu einer stabilen ACE2 Dimerstrukturen führt. Es ist vor allem die C-terminale Domäne des Proteins und dessen post-translationale Modifikationen, welche maßgeblich für die Dimerisierung verantwortlich sind. Um die Besonderheit der ACE2 Dimerstruktur hervorzuheben, wurde die Struktur von membranständigen ACE2 mittels nativem PAGE und ACE2 spezifischem Westernblot analysiert (Fig. 16). In den Spuren A und B sind Zellextrakte (Cortex und Gehirn) sowie in Spur C ein Zellextrakt eines Produktionsklons für ACE2 Dimere, produziert nach Beispiel 1, aufgetragen. Das membranständige Produkt weist hierbei ein deutlich geringeres Molekulargewicht im Vergleich zum Expressionsprodukt nach Beispiel 1 (C und D) auf, obwohl erstes nur aus dem extrazellulären Teil besteht. Dies deutet drauf hin, daß membranständiges ACE2 als Monomer vorliegt. Das Expressionsprodukt hingegen besteht aus Dimeren, die dem Produkt pharmakologische Vorteile verleihen. In Spur D wurde das aufgereinigte finale Produkt analysiert. Dieses weist nur mehr eine einzige Bande eines Molekulargewichtes von ca. 240 kDa auf.

### Beispiel 9: verbesserten pharmakologischen Eigenschaften der ACE2 Dimere

APN 01 ist die Bezeichnung einer physiologischen ACE2 Proteinformulierung, die ausschließlich aus ACE2 Dimerstrukturen besteht. Jeweils zwei ACE2 Monomere bilden hierbei nicht-kovalent aneinander gebundene Komplexe. Die molekularbiologischen Konstrukte, die Expressionszellinie, der Fermentationsprozeß, die Aufreinigung und der Puffer für Lagerung und Applikation sind so selektioniert bzw. konzipiert worden, dass im finalen Produkt ausschließlich stabile ACE2-Dimere vorkommen. Das Dimer aggregiert weder zu größeren Komplexen noch dissoziiert es unter physiologischen Bedingungen zu Monomeren.

In Fig. 15 (Analytisches SEC-HPLC Chromatogramm: APN 01 im Vergleich zu einem Größentrennungsstandard (Bio-RAD GF-Standard, blaue Kurve). Laufbedingungen: Säule: Zorbax GF250, Puffer: 220 mM Na₂HPO₄ + 10% CH₃CN , pH 8.0 bei 1 ml/min). APN 01 eluiert in Form eines einzigen Peaks bei 8,6 Minuten, der sich bezüglich seiner Retentionszeit zwischen Thyroglobulin (680 kDa, Retentionszeit 7,4 Minuten) und bovinem Gamma-globulin (158 kDa, Retentionszeit 8,9 Minuten) befindet. Die berechnete Molekularmasse dieses Komplexes beträgt ca. 214 kDa. Dies entspricht in etwa dem erwarteten Molekulargewicht von zwei ACE2 Einheiten von jeweils 102 kDa.

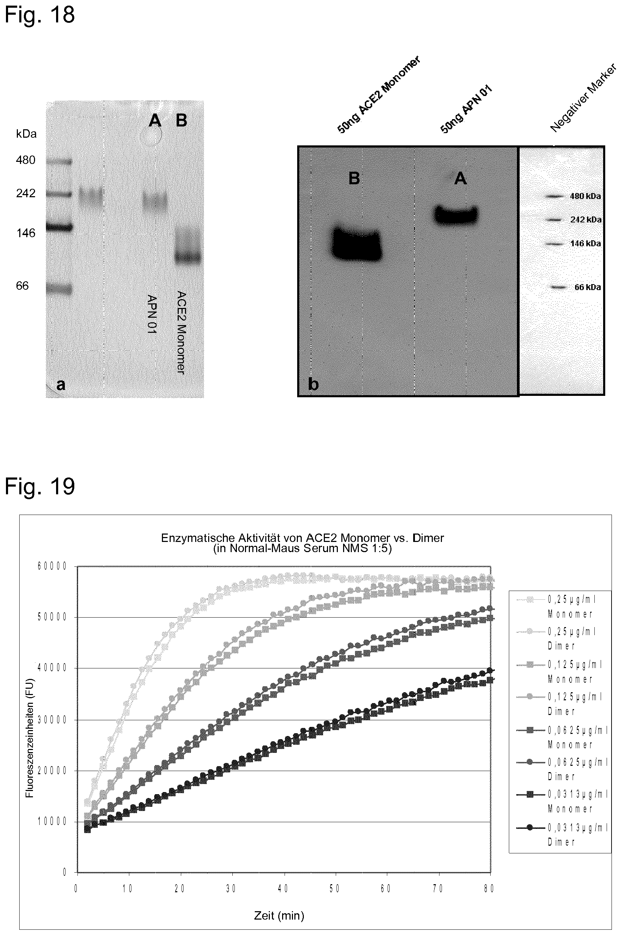

Zu Vergleichszwecken wurde als Referenzmaterial ACE2 Monomere in CHO-Zellen hergestellt, welche ebenfalls mittels SEC-HPLC analysiert wurden. Das diesbezügliche Chromatogramm ist in Fig. 17 dargestellt. Die Monomerform eluiert bei einer Retentionszeit von 9,0 Minuten, was einem Molekulargewicht um 110 kDa entspricht. Beide Produkte wurden ferner mittels PAGE verglichen (siehe Fig. 18a). Während APN 01 (A) eine Proteinbande mit einem Molekulargewicht von ca. 240 kDa aufweist, erscheint die Monomerform (B) bei ca. 100 kDa. Mittels ACE2 spezifischem Western-blot wurde die Identität beider Produkte bestätigt, wie in Fig. 18b ersichtlich ist.

Beide Produkte zeigen eine identische spezifische Enzymaktivität, gemessen anhand eines Aktivitätstests unter Verwendung eines fluoreszenzmarkierten Substrates. Wie in Fig. 19 ersichtlich ist, liegen die Kurven derselben Enzymkonzentration der Monomer- und Dimerform übereinander.

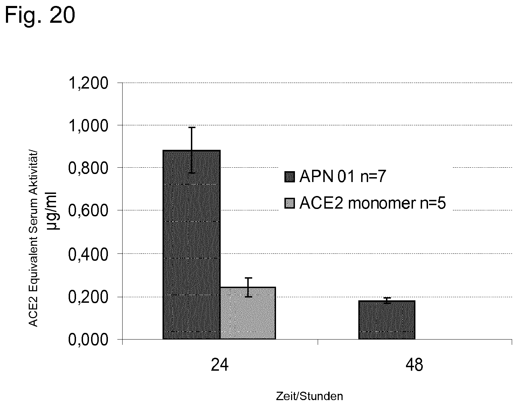

Um die pharmakologischen Eigenschaften beider Produkte zu vergleichen, wurden beide Präparationen BI-6 Mäusen intraperitoneal als Bolusinjektion von 2,5 mg/kg verabreicht und die ACE2 Enzymaktivität in Serumproben nach 24 und 48 Stunden gemessen (siehe Fig. 20). Die Proteinkonzentration wurde so eingestellt, dass alle Tiere jeweils 100 µl einer physiologischen Proteinlösung erhielten. Jeweils 7 Tiere wurden mit APN 01 (ACE2-Dimer) und 5 Tiere mit ACE2 Monomer behandelt. Während vor ACE2 Gabe in keiner Serumprobe ACE2 Aktivität meßbar war, konnte nach der Verabreichung in allen Fällen systemisch ACE2 Aktivität gemessen werden. Bereits 24 Stunden nach Applikation unterschieden sich beide Gruppen signifikant (ttest, p<0,001): Während in den Tieren, die mit dem ACE2-Dimer behandelt wurden, eine Aktivität entsprechend einer ACE2 Konzentration von 0,9 µg/ml gemessen wurde, konnte in der Gruppe, welche ACE2 Monomer erhielt, nur die einer Konzentration von 0,2 µg/ml entsprechende Aktivität gefunden werden. Nach 48 Stunden fanden sich in der Gruppe, die ACE2 Homodimere erhielt, noch 0,2 µg/ml Aktivität, während in der Gruppe, die das Monomer erhielt, keine systemische ACE2 Aktivität mehr nachgewiesen werden konnte. Diese Daten zeigen einen sehr deutlichen pharmakologischen Vorteil der ACE2 Dimerform.

## Patentansprüche

1. Rekombinantes ACE2 Polypeptid, welches als Dimer vorliegt.

2. Rekombinantes ACE2 Polypeptid nach Anspruch 1, **dadurch gekennzeichnet, dass** es glykosyliert ist, wobei die Glycogruppen eines ACE2 Polypeptid Monomers in Summe mindestens 14 Sialylsäure-Reste aufweisen.

3. Rekombinantes ACE2 Polypeptid nach Anspruch 2, **dadurch gekennzeichnet, dass** es an mindestens 80% der möglichen N-Glykosylierungspositionen glykosyliert ist und einen Zuckeranteil von größer als 10% (Massen-%, bezogen auf das gesamte ACE2-Molekül), vorzugsweise größer als 15%, insbesondere größer als 20 %, aufweist.

4. Rekombinantes ACE2 Polypeptid nach Anspruch 1, 2 oder 3, **dadurch gekennzeichnet, dass** mindestens 70% der glykosylierten N-Glykosylierungspositionen eine Struktur unabhängig voneinander ausgewählt aus den Formeln 1-8: 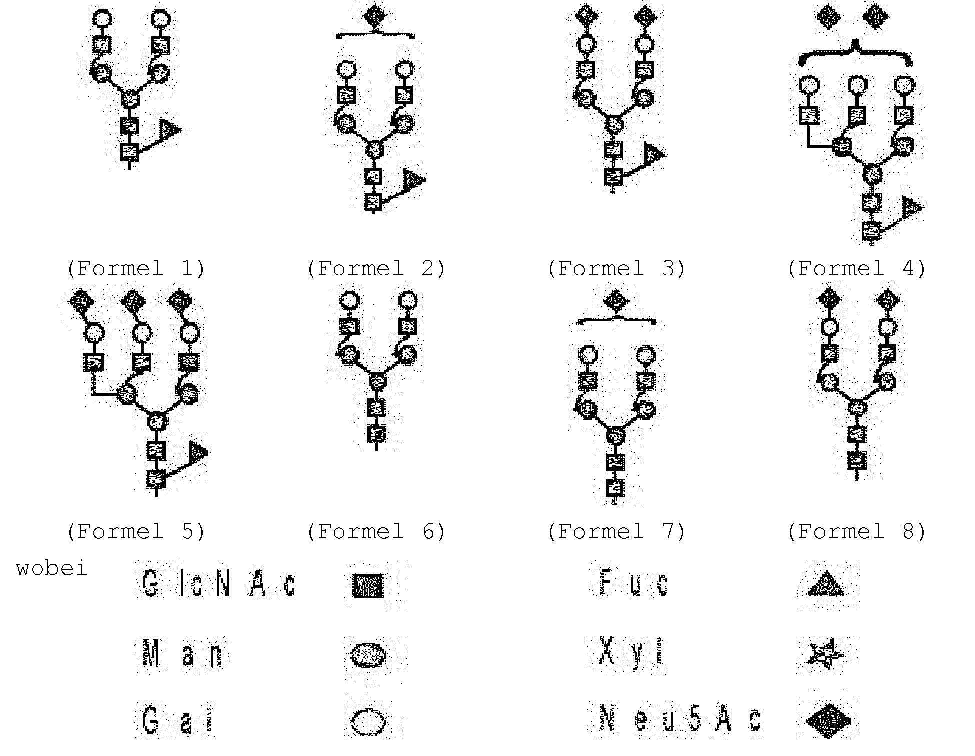 ist, aufweisen.

5. Rekombinantes ACE2 Polypeptid nach einem der Ansprüche 1 bis 4, **dadurch gekennzeichnet, dass** alle der möglichen N-Glykosylierungspositionen glykosyliert sind.

6. Rekombinantes ACE2 Polypeptid nach einem der Ansprüche 1 bis 5, **dadurch gekennzeichnet, dass** mindestens 80%, vorzugsweise mindestens 90%, insbesondere 100%, der glykosylierten N-Glykosylierungspositionen eine Struktur der Formeln 1-8 aufweisen.

7. Rekombinantes ACE2 Polypeptid nach einem der Ansprüche 1 bis 6, **dadurch gekennzeichnet, dass** das eine ACE2 Monomereinheit des Dimers ein Molekulargewicht von mindestens 90 kDa, vorzugsweise mindestens 92 kDa, besonders bevorzugt mindestens 94 kDa, insbesondere mindestens 96 kDa, speziell bevorzugt mindestens 98 kDa, am meisten bevorzugt mindestens 101 kDa, aufweist.

8. Rekombinantes ACE2 Polypeptid nach einem der Ansprüche 1 bis 7, **dadurch gekennzeichnet, dass** das ACE2 Polypeptid keine transmembrane Domäne aufweist, vorzugsweise dass die ACE2 Polypeptidkette aus den Aminosäuren 18-740 von SEQ ID NO: 1 oder enzymatisch aktiven Fragmenten davon besteht.

9. Rekombinantes ACE2 Polypeptid nach einem der Ansprüche 1 bis 8, **dadurch gekennzeichnet, dass** ein zu Ser740 der SEQ ID NO: 1 korrespondierendes Serin O-glykosyliert ist.

10. Rekombinantes ACE2 Polypeptid nach einem der Ansprüche 1 bis 9, **dadurch gekennzeichnet, dass** mind. 70%, vorzugsweise mind. 80%, insbesondere mind. 90%, am meisten bevorzugt 100% der glykosylierten N-Glykosylierungsstellen Sialinsäure aufweisen, vorzugsweise sind die N-Glykosylierungsstellen entsprechend Asn53, Asn90, Asn103, Asn322, Asn432, Asn546, Asn690 der SEQ ID NO: 1 sialysiert.

11. Rekombinantes ACE2 Polypeptid nach einem der Ansprüche 1 bis 10, **dadurch gekennzeichnet, dass** mind. 30%, vorzugsweise mind. 40%, insbesondere mind. 55%, am meisten bevorzugt mind. 70% der glykosylierten N-Glykosylierungsstellen mindestens zwei Sialinsäuren aufweisen.

12. Rekombinantes ACE2 Polypeptid nach einem der Ansprüche 1 bis 11, **dadurch gekennzeichnet, dass** ein zu Asn53 der SEQ ID NO: 1 korrespondierendes Asparagin N-glykosyliert ist, vorzugsweise mit einem Glykan der Strukturen nach Formel 1, 2, 3, 4, 5, 6, 7 oder 8 wie in Anspruch 4 definiert.

13. Rekombinantes ACE2 Polypeptid nach einem der Ansprüche 1 bis 12, **dadurch gekennzeichnet, dass** ein zu Asn90 der SEQ ID NO: 1 korrespondierendes Asparagin N-glykosyliert ist, vorzugsweise mit einem Glykan der Strukturen nach Formel 1, 2, 3, 4, 5, 6, 7 oder 8 wie in Anspruch 4 definiert.

14. Rekombinantes ACE2 Polypeptid nach einem der Ansprüche 1 bis 13, **dadurch gekennzeichnet, dass** ein zu Asn103 der SEQ ID NO: 1 korrespondierendes Asparagin N-glykosyliert ist, vorzugsweise mit einem Glykan der Strukturen nach Formel 1, 2, 3, 4, 5, 6, 7 oder 8 wie in Anspruch 4 definiert.

15. Rekombinantes ACE2 Polypeptid nach einem der Ansprüche 1 bis 14, **dadurch gekennzeichnet, dass** ein zu Asn322 der SEQ ID NO: 1 korrespondierendes Asparagin N-glykosyliert ist, vorzugsweise mit einem Glykan der Strukturen nach Formel 1, 2, 3, 4, 5, 6, 7 oder 8 wie in Anspruch 4 definiert.


SEQUENCE LISTING

<110>  Apeiron Biologics Forschungs- und Entwicklungs GmbH

<120>  ACE2 Polypeptid

<130>  r52100

<150>  AT A 913/2007
<151>  2007-06-12

<150>  EP 08450052.9
<151>  2008-04-08

<160>  1

<170>  PatentIn version 3.5

<210>  1
<211>  740
<212>  PRT
<213>  homo sapiens

<400>  1

Met Ser Ser Ser Ser Trp Leu Leu Leu Ser Leu Val Ala Val Thr Ala
1               5                   10                  15

Ala Gln Ser Thr Ile Glu Glu Gln Ala Lys Thr Phe Leu Asp Lys Phe
            20                  25                  30

Asn His Glu Ala Glu Asp Leu Phe Tyr Gln Ser Ser Leu Ala Ser Trp
        35                  40                  45

Asn Tyr Asn Thr Asn Ile Thr Glu Glu Asn Val Gln Asn Met Asn Asn
    50                  55                  60

Ala Gly Asp Lys Trp Ser Ala Phe Leu Lys Glu Gln Ser Thr Leu Ala
65                  70                  75                  80

Gln Met Tyr Pro Leu Gln Glu Ile Gln Asn Leu Thr Val Lys Leu Gln
                85                  90                  95

Leu Gln Ala Leu Gln Gln Asn Gly Ser Ser Val Leu Ser Glu Asp Lys
            100                 105                 110

Ser Lys Arg Leu Asn Thr Ile Leu Asn Thr Met Ser Thr Ile Tyr Ser
        115                 120                 125

Thr Gly Lys Val Cys Asn Pro Asp Asn Pro Gln Glu Cys Leu Leu Leu
    130                 135                 140

Glu Pro Gly Leu Asn Glu Ile Met Ala Asn Ser Leu Asp Tyr Asn Glu
145                 150                 155                 160

Arg Leu Trp Ala Trp Glu Ser Trp Arg Ser Glu Val Gly Lys Gln Leu
                165                 170                 175

Arg Pro Leu Tyr Glu Glu Tyr Val Val Leu Lys Asn Glu Met Ala Arg
            180                 185                 190

Ala Asn His Tyr Glu Asp Tyr Gly Asp Tyr Trp Arg Gly Asp Tyr Glu
        195                 200                 205

Val Asn Gly Val Asp Gly Tyr Asp Tyr Ser Arg Gly Gln Leu Ile Glu
    210                 215                 220

Asp Val Glu His Thr Phe Glu Glu Ile Lys Pro Leu Tyr Glu His Leu
225                 230                 235                 240

His Ala Tyr Val Arg Ala Lys Leu Met Asn Ala Tyr Pro Ser Tyr Ile
                245                 250                 255

Ser Pro Ile Gly Cys Leu Pro Ala His Leu Leu Gly Asp Met Trp Gly
            260                 265                 270

Arg Phe Trp Thr Asn Leu Tyr Ser Leu Thr Val Pro Phe Gly Gln Lys
        275                 280                 285

Pro Asn Ile Asp Val Thr Asp Ala Met Val Asp Gln Ala Trp Asp Ala
    290                 295                 300

Gln Arg Ile Phe Lys Glu Ala Glu Lys Phe Phe Val Ser Val Gly Leu
305                 310                 315                 320

Pro Asn Met Thr Gln Gly Phe Trp Glu Asn Ser Met Leu Thr Asp Pro
                325                 330                 335

Gly Asn Val Gln Lys Ala Val Cys His Pro Thr Ala Trp Asp Leu Gly
            340                 345                 350

Lys Gly Asp Phe Arg Ile Leu Met Cys Thr Lys Val Thr Met Asp Asp
        355                 360                 365

Phe Leu Thr Ala His His Glu Met Gly His Ile Gln Tyr Asp Met Ala
    370                 375                 380

Tyr Ala Ala Gln Pro Phe Leu Leu Arg Asn Gly Ala Asn Glu Gly Phe
385                 390                 395                 400

His Glu Ala Val Gly Glu Ile Met Ser Leu Ser Ala Ala Thr Pro Lys

                405                 410                 415

His Leu Lys Ser Ile Gly Leu Leu Ser Pro Asp Phe Gln Glu Asp Asn
            420                 425                 430

Glu Thr Glu Ile Asn Phe Leu Leu Lys Gln Ala Leu Thr Ile Val Gly
        435                 440                 445

Thr Leu Pro Phe Thr Tyr Met Leu Glu Lys Trp Arg Trp Met Val Phe
    450                 455                 460

Lys Gly Glu Ile Pro Lys Asp Gln Trp Met Lys Lys Trp Trp Glu Met
465                 470                 475                 480

Lys Arg Glu Ile Val Gly Val Val Glu Pro Val Pro His Asp Glu Thr
                485                 490                 495

Tyr Cys Asp Pro Ala Ser Leu Phe His Val Ser Asn Asp Tyr Ser Phe
            500                 505                 510

Ile Arg Tyr Tyr Thr Arg Thr Leu Tyr Gln Phe Gln Phe Gln Glu Ala
        515                 520                 525

Leu Cys Gln Ala Ala Lys His Glu Gly Pro Leu His Lys Cys Asp Ile
    530                 535                 540

Ser Asn Ser Thr Glu Ala Gly Gln Lys Leu Phe Asn Met Leu Arg Leu
545                 550                 555                 560

Gly Lys Ser Glu Pro Trp Thr Leu Ala Leu Glu Asn Val Val Gly Ala
                565                 570                 575

Lys Asn Met Asn Val Arg Pro Leu Leu Asn Tyr Phe Glu Pro Leu Phe
            580                 585                 590

Thr Trp Leu Lys Asp Gln Asn Lys Asn Ser Phe Val Gly Trp Ser Thr
        595                 600                 605

Asp Trp Ser Pro Tyr Ala Asp Gln Ser Ile Lys Val Arg Ile Ser Leu
    610                 615                 620

Lys Ser Ala Leu Gly Asp Lys Ala Tyr Glu Trp Asn Asp Asn Glu Met
625                 630                 635                 640

Tyr Leu Phe Arg Ser Ser Val Ala Tyr Ala Met Arg Gln Tyr Phe Leu
                645                 650                 655

Lys Val Lys Asn Gln Met Ile Leu Phe Gly Glu Glu Asp Val Arg Val
            660                 665                 670

Ala Asn Leu Lys Pro Arg Ile Ser Phe Asn Phe Phe Val Thr Ala Pro
        675                 680                 685

Lys Asn Val Ser Asp Ile Ile Pro Arg Thr Glu Val Glu Lys Ala Ile
    690                 695                 700

Arg Met Ser Arg Ser Arg Ile Asn Asp Ala Phe Arg Leu Asn Asp Asn
705                 710                 715                 720

Ser Leu Glu Phe Leu Gly Ile Gln Pro Thr Leu Gly Pro Pro Asn Gln
                725                 730                 735

Pro Pro Val Ser
            740